Europäisches Patentamt

European Patent Office

Office européen des brevets

(11) Publication number: **0 327 009 B1**

# EUROPEAN PATENT SPECIFICATION

(45) Date of publication of patent specification: **13.07.94**

(51) Int. Cl.5: **C12N 1/20**, C12P 1/06, A61K 35/66, //(C12N1/20, C12R1:465),(C12P1/06, C12R1:465)

(21) Application number: **89101607.3**

(22) Date of filing: **31.01.89**

(54) WS-9326A, WS-9326B and their derivatives.

(30) Priority: **02.02.88 GB 8802229**

(43) Date of publication of application:
**09.08.89 Bulletin 89/32**

(45) Publication of the grant of the patent:
**13.07.94 Bulletin 94/28**

(84) Designated Contracting States:
**AT BE CH DE ES FR GB GR IT LI LU NL SE**

(56) References cited:
EP-A- 0 235 795
FR-A- 91 781

CHEMICAL ABSTRACTS, vol. 85, 1976, page 495, abstract no. 18903m, Columbus,Ohio, US; H. DRAUTZ et al.

JOURNAL OF ANTIBIOTICS, vol. XLI, no. 10, October 1988, pages 1300-1315, Japan Antibiotics Research Association, Tokyo, JP; J.E. Hochlowski et al.

(73) Proprietor: **FUJISAWA PHARMACEUTICAL**

CO., LTD.
3, Doshomachi 4-chome
Higashi-ku
Osaka-shi Osaka 541(JP)

(72) Inventor: **Kino, Tohru**
11-11, Nakamuraminami 6-chome
Tsuchiura-shi Ibaraki 300(JP)
Inventor: **Nishikawa, Motoaki**
5-4-405, Umezono 2-chome
Tsukuba-shi Ibaraki 305(JP)
Inventor: **Ezaki, Masami**
17-1-304, Namiki 3-chome
Tsukuba-shi Ibaraki 305(JP)
Inventor: **Kiyoto, Sumio**
17-1-405, Namiki 3-chome
Tsukuba-shi Ibaraki 305(JP)
Inventor: **Okuhara, Masakuni**
14-10, Umezono 2-chome
Tsukuba-shi Ibaraki 305(JP)
Inventor: **Takase, Shigehiro**
15-2-205, Umezono 2-chome
Tsukuba-shi Ibaraki 305(JP)

Inventor: **Okada, Satoshi**
**13-1, Namiki 4-chome**
**Tsukuba-shi Ibaraki 305(JP)**
Inventor: **Shigematsu, Nobuharu**
**5-4-601, Umezono 2-chome**
**Tsukuba-shi Ibaraki 305(JP)**

(74) Representative: **Türk, Gille, Hrabal, Leifert**
**Brucknerstrasse 20**
**D-40593 Düsseldorf (DE)**

**Description**

This invention relates to novel WS-9326A, WS-9326B, derivatives thereof and pharmaceutically acceptable salts thereof which have pharmacological activities. More particularly, it relates to novel WS-9326A, WS-9326B, derivatives thereof and pharmaceutically acceptable salts thereof which have pharmacological activities such as substance P antagonism, neurokinin A (substance K) antagonism or the like, to process for their production and to a pharmaceutical composition containing the same.

Accordingly, one object of this invention is to provide the WS-9326A, WS-9326B, their derivatives and pharmaceutically acceptable salts thereof which are useful for treatment and prevention of asthma and the like.

Another object of this invention is to provide processes for production of the WS-9326A, WS-9326B, their derivatives and pharmaceutically acceptable salts thereof.

A further object of this invention is to provide a pharmaceutical composition containing, as an active ingredient, the WS-9326A, WS-9326B, their derivatives or pharmaceutically acceptable salts thereof.

Still further object of this invention is to provide a use of the WS-9326A, WS-9326B, their derivatives and pharmaceutically acceptable salts thereof for the treatment and prevention of asthma and the like.

The WS-9326A and WS-9326B of this invention can be produced by fermentation of a WS-9326A and/or WS-9326B-producing strain belonging to the genus Streptomyces such as Streptomyces violaceoniger No. 9326 in a nutrient medium.

Particulars of microorganism used for the production of the WS-9326A and WS-9326B will be explained in the following.


THE MICROORGANISM


The microorganism which can be used for the production of the WS-9326A and WS-9326B is a WS-9326A and/or WS-9326B-producing strain belonging to the genus Streptomyces, among which Streptomyces violaceoniger No. 9326 has been newly isolated from a soil sample collected at Suwa City, Nagano Prefecture, Japan.

A lyophilized sample of the newly isolated Streptomyces violaceoniger No. 9326 has been deposited with the Fermentation Research Institute, Agency of Industrial Science and Technology (1-3, Higashi 1-chome, Tsukuba-shi, Ibaraki-ken 305, Japan) under the number of FERM BP-1667 (deposited date : January 20, 1988).

It is to be understood that the production of the novel WS-9326A and WS-9326B is not limited to the use of the particular organism described herein, which is given for the illustrative purpose only. This invention also includes the use of any mutants which are capable of producing the WS-9326A and WS-9326B including natural mutants as well as artificial mutants which can be produced from the described organism by conventional means such as irradiation of X-rays, ultra-violet radiation, treatment with N-methyl-N′-nitro-N-nitrosoguanidine, 2-aminopurine, and the like.

The Streptomyces violaceoniger No. 9326 has the following morphological, cultural, biological and physiological characteristics.


[1] Morphological Characteristics :


The methods described by Shirling and Gottlieb (Shirling, E.B. and D. Gottlieb : Methods for characterization of Streptomyces species. International Journal of Systematic Bacteriology, 16, 313 - 340, 1966) were employed for this taxonomic study.

Morphological observations were made with light and electron microscopes on cultures grown at 30°C for 14 days on oatmeal agar, yeast-malt extract agar and inorganic salts-starch agar.

The vegetative mycelium developed well without fragmentation. The aerial mycelium branched monopodially and formed spiral chains of spores with 10 to 30 spores per chain. The spores had a smooth surface and were oval in shape with a size of 0.6-0.8 x 0.8-1.3 $\mu$m. Sclerotic granules, sporangia and zoospores were not observed.


[2] Cultural Characteristics :


Cultural characteristics were observed on ten kinds of media described by Shirling and Gottlieb as mentioned above, and by Waksman (Waksman, S.A. : The actinomycetes, Vol. 2 : Classification, identification and description of genera and species. The williams and Wilkins Co., Baltimore, 1961).

The incubation was carried out at 30°C for 21 days. The color names used in this study were taken from Methuen Handbook of Colour (Kornerup, A. and J.H. Wanscher : Methuen Handbook of Colour, Methuen, London, 1978). The results are shown in Table 1.

Table 1.  Cultural characteristics of strain No. 9326

| Medium | | Cultural characteristics |
|---|---|---|
| yeast-malt extract agar | G : | good |
| | A : | abundant, brownish gray (6E2) |
| | R : | dark brown (7F6) |
| | S : | none |
| oatmeal agar | G : | good |
| | A : | moderate, dark brown(7E3) |
| | R : | brownish gray (7F2) |
| | S : | none |
| inorganic salts-starch agar | G : | good |
| | A : | abundant, brownish gray (7E2) |
| | R : | yellowish brown (5E6) |
| | S : | none |
| glycerin-asparagine agar | G : | good |
| | A : | abundant, grayish violet (19E3) |
| | R : | brown (6E4) |

| | | | |
|---|---|---|---|
| | S : | none | |
| peptone-yeast extract-iron agar | G : | good | |
| | A : | thin, grayish white (1B1) | |
| | R : | yellowish brown (5D6) | |
| | S : | none | |
| tyrosine agar | G : | good | |
| | A : | abundant, brownish gray (9E2) | |
| | R : | brown (6E5) to black | |
| | S : | none | |
| glucose-asparagine agar | G : | good | |
| | A : | moderate, bluish gray (19E2) | |
| | R : | brown (6F4) | |
| | S : | none | |
| nutrient agar | G : | moderate | |
| | A : | moderate, brownish gray (9E2) | |
| | R : | grayish brown (5E3) to yellowish brown (5F4) | |
| | S : | none | |
| Bennet agar | G : | poor | |
| | A : | poor, dark brown (6F4) | |
| | R : | dark brown (6F4) | |
| | S : | none | |
| sucrose-nitrate agar | G : | poor | |
| | A : | none | |
| | R : | grayish brown (6E3) | |
| | S : | none | |

Abbreviation :   G=growth,   A=aerial mycelium,
R=reverse side color,   S=soluble pigment

The aerial mycelium was gray to brownish gray. Part of colony became black and moist, and showed hygroscopic character on most agar media. Reverse side of growth was yellowish brown, brown and dark brown. Reverse mycelium pigment was not pH sensitive. Melanoid pigments and other soluble pigments were not produced.

The cell wall analysis was performed by the methods of Becker et al. (Becker, B., M. P. Lechevalier, R. E. Gordon and H. A. Lechevalier : Rapid differentiation between Nocardia and Streptomyces by paper chromatography of whole cell hydrolysates : Appl. Microbiol., 12, 421-423, 1964) and Yamaguchi (Yamaguchi, T. : Comparison of the cell wall composition of morphologically distinct actinomycetes : J. Bacteriol., 89, 444-453, 1965). Analysis of whole cell hydrolysates of strain No. 9326 showed the presence of LL-diaminopimelic acid. Accordingly, the cell wall of this strain is believed to be of type I.

[3] Biological and Physiological Properties :

Physiological properties and utilization of carbon sources are shown in Table 2 and 3, respectively.

Utilization of carbon sources was examined according to the methods of Pridham and Gottlieb (Pridham, T. G. and D. Gottlieb : The utilization of carbon compounds by some Actinomycetales as an aid for species determination : J. Bacteriol., 56, 107-114, 1948).

Table 2

| Physiological properties of strain No. 9326 | |
|---|---|
| Conditions | Characteristics |
| temperature range for growth | 11 ° C - 47 ° C |
| optimum temperature range for growth | 29 ° C - 31 ° C |
| gelatin liquefaction | positive |
| milk coagulation | negative |
| milk peptonization | positive |
| starch hydrolysis | positive |
| production of melanoid pigment | negative |
| decomposition of cellulose | negative |

Table 3.  Carbon utilization of strain No. 9326

| Compounds | Growth | |
|---|---|---|
| D-glucose | + | |
| sucrose | + | |
| D-xylose | + | |
| D-fructose | + | |
| L-rhamnose | + | |
| raffinose | + | + :  utilization |
| L-arabinose | + | |
| inositol | + | |
| mannitol | + | |

The morphology and chemical characteristics of strain No. 9326 permitted a clear assignment of the organism to the genus Streptomyces. Strain No. 9326 was compared with Streptomyces species described in the 8th edition of Bergey's manual (Buchanan, R.E. and N.E. Gibbons : Bergey's manual of determinative bacteriology, eight edition. The Williams and Wilkins Co., Baltimore, 1974), Streptomyces species described in Shirling's ISP reports [(Shirling, E. B. and D. Gottlieb : Cooperative description of type culture of Streptomyces.2. Species descriptions from first study. Intern. J. Syst. Bacteriol. 18 : 69-189, 1968), (Shirling, E.B. and D. Gottlieb : Cooperative description of type culture of Streptomyces.3. Additional species descriptions from first and second studies. Intern. J. Syst. Bacteriol. 18 : 279-392, 1968) and (Shirling, E.B. and D. Gottlieb : Cooperative description of type culture of Streptomyces.4. Species descriptions fron the second, third and fourth studies. Intern. J. Syst. Bacteriol. 19: 391-512, 1969)], the species listed on "Approved lists of bacterial names" (Skerman, V.B.D.; V. McGowan & P.H.A. Sneath : Approved list of bacterial names. Intern. J. Syst. Bacteriol. 30: 225-420, 1980) and the species described in the other references [(Williams, S.T. : M. Goodfellow, G. Alderson, E.M.H. Wellington, P.H.A. Sneath and M.J. Sackin : Numerical classification of Streptomyces and related genera. J. Gen. Microbiol. 129: 1743-1813, 1983) and (Dietz, A. : Criteria for characterization of Hygroscopicus strains. In "Actinomycetes; The Boundary Microorganisms" pp183-191 Edited by T. Arai, 1976)].

As a result, it was found that strain No. 9326 proved to closely resemble Streptomyces violaceoniger. Therefore, strain No. 9326 was identified as Streptomyces violaceoniger and designated Streptomyces violaceoniger No.9326.

PRODUCTION OF WS-9326A AND WS-9326B

The novel WS-9326A and WS-9326B of this invention can be produced by culturing a WS-9326A and/or WS-9326B-producing strain belonging to the genus Streptomyces (e.g. Streptomyces violaceoniger No.9326, FERM BP-1667) in a nutrient medium.

In general, the WS-9326A and WS-9326B can be produced by culturing the WS-9326A and/or WS-9326B-producing strain in an aqueous nutrient medium containing sources of assimilable carbon and nitrogen, preferable under aerobic conditions (e.g. shaking culture, submerged culture, etc.).

The preferred sources of carbon in the nutrient medium are carbohydrates such as glucose, xylose, galactose, glycerin, starch, dextrin, and the like.

Other sources which may be included are maltose, rhamnose, raffinose, arabinose, mannose, salicin, sodium succinate, and the like.

The preferred sources of nitrogen are yeast extract, peptone, gluten meal, cottonseed meal, soybean meal, corn steep liquor, dried yeast, wheat germ, feather meal, peanut powder etc., as well as inorganic and organic nitrogen compounds such as ammonium salts (e.g. ammonium nitrate, ammonium sulfate, ammonium phosphate, etc.), urea, amino acid, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts and the like. If necessary, especially when the culture medium foams seriously, a defoaming agent such as liquid paraffin, fatty oil, plant oil, mineral oil or silicone may be added.

As the conditions for the production of the WS-9326A and WS-9326B in massive amounts, submerged aerobic cultural conditions are preferred therefor. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production of the WS-9326A and WS-9326B. Accordingly, it is desirable first to produce a vegetative inoculum of the organism by inoculating a relatively small quantity of culture medium with spores or mycelia of the organism and culturing said inoculated medium, and then to transfer the cultured vegetative inoculum aseptically to large tanks. The medium, in which the vegetative inoculum is produced, is substantially the same as or different from the medium utilized for the production of the WS-9326A and WS-9326B.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 20°C and 40°C, preferably 25-35°C, for a period of about 50 hours to 150 hours, which may be varied according to fermentation conditions and scales.

Thus produced WS-9326A and WS-9326B can be recovered from the culture medium by conventional means which are commonly used for the recovery of other known biologically active subtances. The WS-9326A and WS-9326B produced are found in the cultured filtrate and mycelium, and accordingly the WS-9326A and WS-9326B can be isolated and purified from the filtrate and the mycelium, which are obtained by filtering or centrifuging the cultured broth, by a conventional method such as concentration under reduced pressure, lyophilization, extraction with a conventional solvent, pH adjustment, treatment with a conventional resin (e.g. anion or cation exchange resin, non-ionic adsorption resin, etc.), treatment with a conventional absorbent (e.g. activated charcoal, silicic acid, silica gel cellulose, alumina, etc.), crystallization, recrystallization, and the like.

The WS-9326A produced according to the aforementioned process possesses the following physical and chemical properties.

(1) Form and Color :
    colorless powder

(2) Color Reaction :
    Positive :    cerium sulfate reaction, iodine vapor reaction, ferric chloride-potassium ferricyanide reaction,
    Negative :    ninhydrine reaction, Molish reaction, ferric chloride reaction, Ehrlich reaction, Pauli reaction

(3) Solubility :
    Soluble :    methanol, ethanol
    Sparingly Soluble :    acetone, ethyl acetate
    Insoluble :    water, chloroform

(4) Melting Point : 187-190°C

(5) Specific Rotation :
    $[\alpha]_D^{23}$ : -84° (C = 1.0, MeOH)

(6) Ultraviolet Absorption Spectrum :

$$\lambda_{max}^{MeOH} = 280nm\ (\varepsilon=34,700)$$

(7) Infrared Absorption Spectrum :

$$\nu_{max}^{KBr} = 3300, 3050, 2950, 2920, 2860, 1730, 1650,$$
$$1610, 1560, 1540, 1530, 1510, 1440, 1380,$$
$$1340, 1280, 1240, 1170, 1110, 1080, 1060,$$
$$1040, 970, 920, 880, 860, 830\ cm^{-1},$$

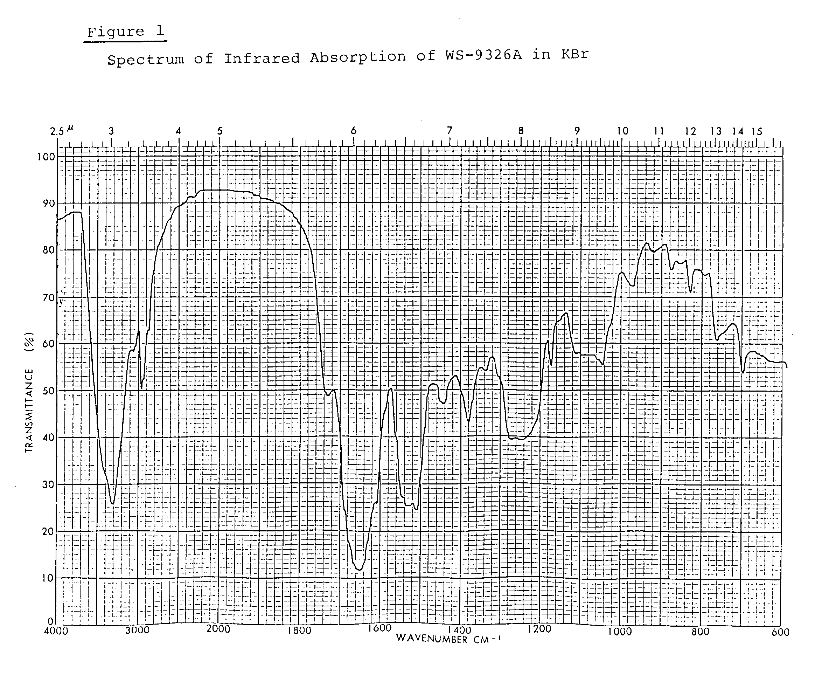

the chart of which is shown in Figure 1,

(8)

| Elementary Analysis : | | | |
|---|---|---|---|
| Found : | C 60.18, | H 6.61, | N 10.32 |
| Calcd. for $C_{54}H_{68}N_8O_{13} \cdot 2H_2O$ : | C 60.43, | H 6.76, | N 10.44 |

(9) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate (Merck) | methanol-water (8:2, V/V) | 0.46 |

(10) Molecular Formula : $C_{54}H_{68}N_8O_{13}$

(11) Molecular Weight :

FAB-MS : m/z 1037 $(M + H)^+$

(12) Property of the Substance :

acidic substance

(13) $^{13}$C Nuclear Magnetic Resonance Spectrum : (100 MHz, $CD_3OD$) $\delta$

| | |
|---|---|
| 175.69 (s), | 174.70 (s), |
| 173.73 (s), | 173.38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q), |

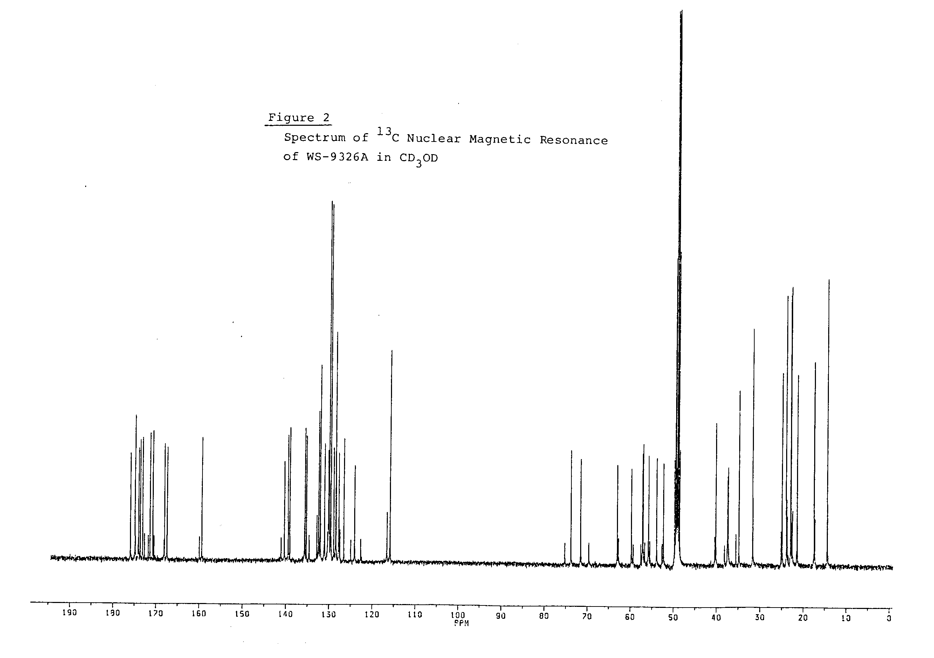

the chart of which is shown in Figure 2,

(14) $^1$H Nuclear Magnetic Resonance Spectrum : (400 MHz, $CD_3OD$) $\delta$

| | |
|---|---|
| 7.80 | (1H, d, J = 8Hz), |
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 and 7Hz), |
| 5.55 | (1H, m), |
| 5.35 | (1H, broad signal), |
| 5.10 | (1H, dd, J = 3 and 9.5Hz), |
| 4.68 | (1H, d, J = 10Hz), |
| 4.55 | (1H, t, J = 6Hz), |
| 4.48 | (1H, dd, J = 3 and 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 and 14Hz), |
| 2.94 | (1H, dd, J = 3 and 16Hz), |
| 2.89 | (3H, s), |
| 2.74 | (1H, dd, J = 9.5 and 16Hz), |
| 2.69 | (1H, dd, J = 12 and 14Hz), |
| 2.14 | (2H, m), |
| 1.5-1.4 | (2H, m), |
| 1.20 | (3H, d, J = 6Hz), |
| 1.08 | (3H, d, J = 6Hz), |
| 1.0-0.8 | (2H, m) |
| 0.91 | (3H, t, J = 7Hz), |
| 0.6 | (1H, m), |
| 0.53 | (3H, d, J = 6Hz), |
| 0.51 | (3H, d, J = 6Hz), |

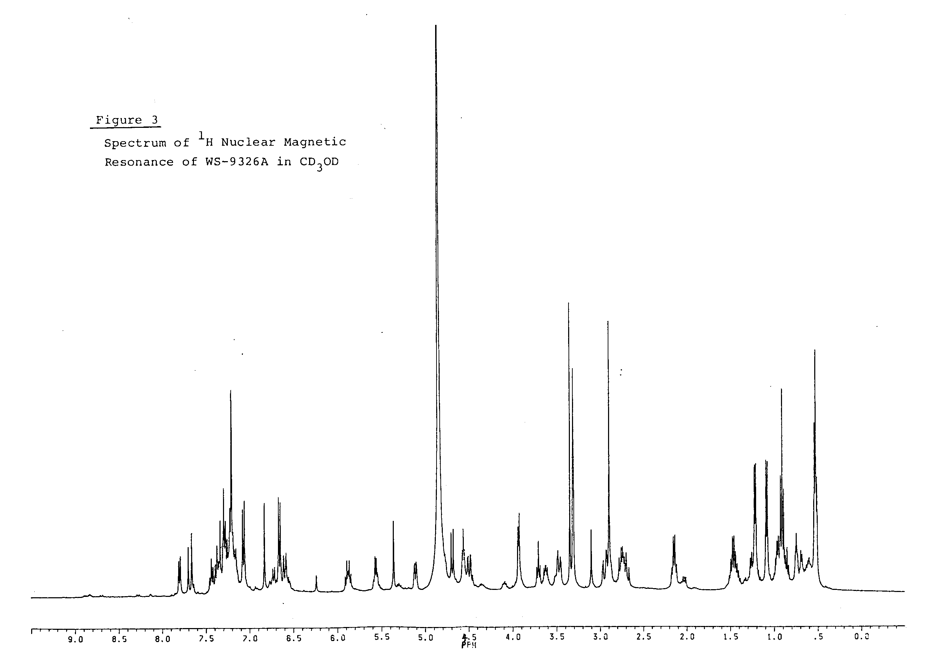

the chart of which is shown in Figure 3,

(15) Amino-Acid Analysis :

WS-9326A (5 mg) was hydrolyzed at 110°C for 20 hours with hydrochloric acid (2 ml) in a sealed tube. The mixture was evaporated to dryness to give the hydrolysis products which were analyzed on a Hitachi 835 automatic amino-acid analyzer.

The results of the amino acid analysis :

Thr (2), Leu (1), Phe (1), Asp (1), Ser (1), methylamine (1) and ammonia (1)

With regard to the WS-9326A, it is to be noted that $^{13}$C and $^1$H Nuclear Magnetic Resonance Spectra shown in Figures 2 and 3 show that the WS-9326A exists in at least two stable conformations in $CD_3OD$ solution and the chemical shifts described in the above (13) and (14) are those of the major conformer of WS-9326A.

The WS-9326B produced according to the aforementioned process possesses the following physical and chemical properties.

(1) Form and Color : colorless amorphous powder

(2) Color Reaction :

    Positive :      cerium sulfate reaction, iodine vapor reaction

    Negative :     ninhydrine reaction

(3) Solubility :

    Soluble :             methanol

    Sparingly Soluble :    ethanol

    Insoluble :          water, acetone, ethyl acetate, chloroform

(4) Melting Point : 165-170°C (dec.)

(5) Specific Rotation :

    $[\alpha]_D^{23}$ : -64° (C = 1.0, MeOH)

(6) Ultraviolet Absorption Spectrum :

$$\lambda_{max}^{MeOH} = 283 \text{ nm } (\varepsilon=27,000)$$

(7) Molecular Formula : $C_{54}H_{70}N_8O_{13}$

(8)

| Elemental Analysis : | | | |
|---|---|---|---|
| Found : | C 59.97, | H 6.87, | N 10.29 |
| Calcd. for $C_{54}H_{70}N_8O_{13} \cdot 2H_2O$ : | C 60.32, | H 6.94, | N 10.42 |

(9) Molecular Weight :
    FAB-MS :     m/z 1061.6 $(M + Na)^+$

(10) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.25 |

(11) Infrared Absorption Spectrum :

$$\nu_{max}^{KBr} = 3300, \ 3050, \ 2950, \ 1735, \ 1660, \ 1530,$$
$$1510, \ 1450, \ 1400, \ 1380, \ 1340, \ 1260,$$
$$1220, \ 1080, \ 980, \ 920 \text{ cm}^{-1}$$

(12) $^{13}$C Nuclear Magnetic Resonance Spectrum : (100 MHz, $CD_3OD$) $\delta$

| | |
|---|---|
| 174.99 (s), | 174.54 (s), |
| 173.60 (s) | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q), | |

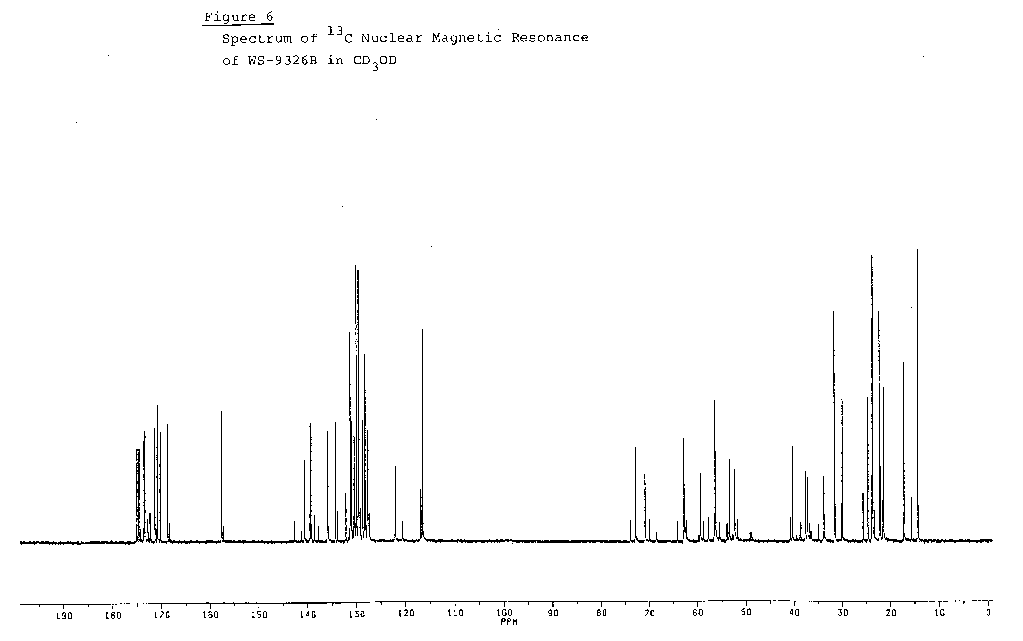

the chart of which is shown in Figure 6
(13) $^1$H Nuclear Magnetic Resonance Spectrum : (400 MHz, $CD_3OD$) $\delta$

| 7.86 | (1H, d, J = 16Hz), |
|---|---|
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 and 11.5Hz), |
| 5.08 | (1H, dd, J = 3.5 and 10Hz), |
| 5.04 | (1H, q, J = 6.5Hz), |
| 4.66 | (1H, dd, J = 3.5 and 13Hz), |
| 4.65 | (1H, d, J = 11.5Hz), |
| 4.56 | (1H, dd, J = 2.5 and 7Hz), |
| 4.48 | (1H, dd, J = 4.5 and 11Hz), |
| 4.46 | (1H, s), |
| 3.88 | (2H, m), |
| 3.64 | (2H, m), |
| 3.51 | (1H, dd, J = 3.5 and 14Hz), |
| 3.17 | (1H, dd, J = 4.5 and 14Hz), |
| 3.01 | (1H, dd, J = 11 and 14Hz), |
| 2.94 | (1H, dd, J = 3.5 and 16Hz), |
| 2.71 | (3H, s), |
| 2.71 | (1H, dd, J = 10 and 16Hz), |
| 2.64 | (1H, dd, J = 13 and 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz), |

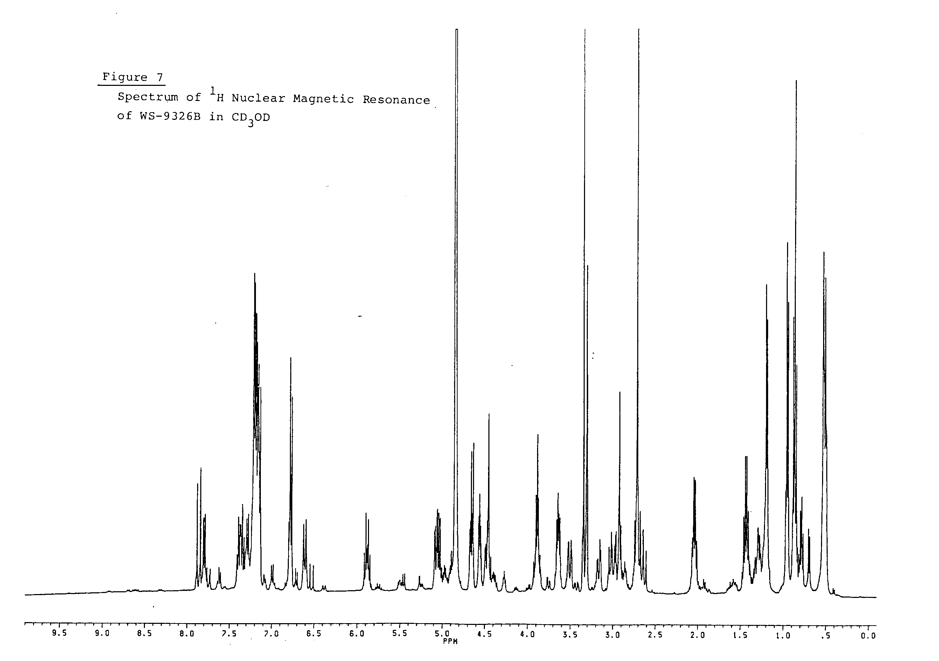

the chart of which is shown in Figure 7.

With regard to the WS-9326B, it is to be noted that $^{13}C$ and $^{1}H$ Nuclear Magnetic Resonance Spectra shown in Figures 6 and 7 show that the WS-9326B exists in at least two stable conformations in $CD_3OD$ solution and the chemical shifts described in the above (12) and (13) are those of the major conformer of WS-9326B.

Suitable "derivatives of the WS-9326A" may include the WS-9326A substituted with 1 to 3 acyl group-(s), and the like.

Suitable "derivatives of the WS-9326B" may include the WS-9326B substituted with 1 to 3 acyl group-(s), and the like.

Suitable "acyl" may include a conventional one such as lower alkanoyl (e.g., formyl, acetyl, propionyl, etc.) or the like.

The WS-9326A substituted with 1 to 3 acyl group(s), WS-9326B substituted with 1 to 3 acyl group(s) or salts thereof can be prepared by reacting the WS-9326A, WS-9326B or salts thereof with acylating agent(s).

This reaction can be carried out in a conventional method such as the method described in Example 2, 4 or 5 as mentioned below, or the like.

Preferred embodiments of derivatives of the WS-9326A or WS-9326B is the WS-9326A subtituted with one acetyl group (hereinafter referred to as monoacetyl-WS-9326A), WS-9326A substituted with two acetyl groups (hereinafter referred to as diacetyl-WS-9326A) or WS-9326A substituted with three acetyl groups (hereinafter referred to as triacetyl-WS-9326A).

Suitable pharmaceutically acceptable salts of WS-9326A, WS-9326B and their derivatives are conventional non-toxic salts.

BIOLOGICAL PROPERTIES OF THE WS-9326A, WS-9326B AND THEIR DERIVATIVES

The WS-9326A, WS-9326B, their derivatives and pharmaceutically acceptable salts thereof possess pharmacological activities such as substance P antagonism, neurokinin A (substance K) antagonism or the like, and therefore are useful for the treatment and prevention of asthma and the like.

As an example for showing such pharmacological activity, some pharmacological test data are illustrated in the following.

(1) Radioligand binding assay

(a) Crude membrane preparation

Brain

Female Wister rats (200 g) were used and all reagents were purchased from Sigma Chemical Company. Whole brains (4 g) were minced into small pieces, and homogenized in 8 volumes of ice cold Medium I (50 mM Tris-HCℓ pH 7.5, 5 mM MnCℓ$_2$, 0.02% BSA, 2$\mu$ g/ml chymostatin, 4 $\mu$g/mℓ leupeptin and 40 $\mu$g/ml bacitracin) with a Ultra-Disperser (Yamato MODEL LK-21). The homogenate was either stored at -20°C or used in binding experiments immediately.

Lung

Male albino Hartley strain guinea pigs (600 g) were sacrificed by decapitation. The trachea and lungs were removed, and stored at -80°C until use. These tissue (150 g) was thawed and homogenized in 500 ml buffer (0.25 M sucrose, 50 mM Tris-HCℓ pH 7.5, 0.1 mM EDTA) with a compact mixer (Matsuden MJ-761). The tissue was homogenized with a Ultra-Disperser (Yamato MODEL LK-21) at a setting of maximum range for 10-s at 10-s intervals with cooling between homogenizations (total homogenization's time : 60 seconds). The homogenate was centrifuged (900 x g for 10 min.) to remove tissue clumps and the supernatant centrifuged at 14000 x g for 20 min. to yield pellets which were referred to as crude membrane fractions. The pellets were resuspended in Medium I, homogenized with a teflon homogenizer and centrifuged at 14000 x g for 20 min. The pellets were stored at -20°C.

(b) $^3$H-substance P binding to preparative membranes

$^3$H-substance P (1nM, New England Nuclear) was incubated with 50 $\mu$ℓ of the membrane preparation in medium I at 4°C for 30 minutes in a final volume of 250 $\mu$ℓ. At the end of the incubation period, its contents were quickly filtered over a whatmann GF/B glass fibre filter (pretreated with 0.1% polyethyleneimine for 3 hour prior to use) using cell harvester (Brandel M-24S). The filters were then washed ten times with a total of 3 ml of the washing buffer (50 mM Tris-HCℓ pH 7.5) at 0°C. The radioactivity was counted in 3 ml of Aquazol-2 in Packard sintillation counter (Packard TRI-CARB 4530).

Table 4

| WS-9326A, WS-9326B or triacetyl-WS-9326A displacement of specific [$^3$H] substance P binding to rat brain and guinea pig lung membranes. | | |
|---|---|---|
| | $IC_{50}$ (M) | |
| | brain | lung |
| WS-9326A | $2.5 \times 10^{-5}$ | $3.8 \times 10^{-6}$ |
| Triacetyl-WS-9326A | $9.4 \times 10^{-5}$ | $7.7 \times 10^{-5}$ |
| WS-9326B | | $8.8 \times 10^{-5}$ |

(2) Effect of WS-9326A on guinea pig trachea

Tracheal spiral strips were prepared from adult, male, albino Hartley strain guinea pigs (600 g) according to standard technique and placed in jacketed 30 ml glass tissue bath. The tension of tracheal strips was measured isometrically by means of force displacement transducer coupled to a polygraph (Biopysiograph 180 system, San-Ei Instrument). Tracheal strips (2 mm width and 50 mm length) were suspended under a resting tension of 500 mg in 30 ml organ baths containing warm (37°C) oxygenated (95% O$_2$ : 5% CO$_2$) Tyrode solution of following composition : NaCℓ 137 mM (8 g/liter), KCℓ 2.7 mM (0.2

g/liter), $CaCl_2 \cdot 2H_2O$ 1.8 mM (0.264 g/liter), $MgCl_2 \cdot 6H_2O$ 1.02 mM (0.208 g/liter), $NaHCO_3$ 11.9 mM (1g/liter), $NaH_2PO_4 \cdot 2H_2O$ 0.42 mM (0.066 g/liter) and glucose 5.5 mM (1 g/liter). The tissues were equilibrated for 90 minutes and then WS-9326A was tested against various bronchoconistrictor (substance P $10^{-8}$M and neurokinin A $10^{-9}$M). The tension was recorded with a San-Ei Rectigraph-8S recorder (San-Ei Instrument).

Table 5

| Effect of WS-9326A on the contractile responses of guinea pig trachea induced by neurokinin A (NKA) and substance P (SP). | | |
|---|---|---|
| WS-9326A (ug/ml) | Inhibition % | |
| | NKA$10^{-9}$M | SP $10^{-8}$M |
| 3 | 44% | -10% |
| 10 | 79% | 50% |
| 30 | 100% | 63% |
| IC$_{50}$ (M) | $3.5 \times 10^{-6}$ | $9.7 \times 10^{-6}$ |

(3) Effect of WS-9326A on the bronchoconstriction induced by neurokinin A and capsaicin.

Male Hartley strain guinea-pigs weighing 300-500 g were immobilized with sodium pentobarbital (10 mg/animal administered intraperitoneally). The jugular vein was cannulated for administration of neurokinin A (or capsaicin) and drug. A catheter was also intubated into trachea for artifical ventilation. The animal was respirated by means of a miniature respiration pump (Harvard B-34, 5 ml/stroke, 60 strokes/minute). Resistance to lung inflation was measured by a modification of Konzett-Rössler overflow technique.

Agonist was administered iv and the antagonist drug (prepared in 0.1% methyl cellulose-saline) was administered iv as shown below.

15 min 15 min 15 min 15 min 15 min 15 min 15 min

2 min

↑ : agonist (neurokinin A or capsaicin)

↑ : antagonist (WS-9326A)

Table 6

Inhibition of neurokinin A induced bronchoconstriction
by WS-9326A

n : 5

| Dose | Inhibition (%) of neurokinin A (1n mol/kg, iv) response | | | | |
|---|---|---|---|---|---|
| | 2 minutes | 17 minutes | 32 minutes | 47 minutes | 62 minutes |
| 10 mg/kg, iv | -16.5±5.6 | 30.2±12.9 | 45.8±13.8 | 55.4±8.1 | 49.9±13.4 |

Table 7

Inhibition of capsaicin induced bronchoconstriction by WS-9326A

n : 4

| Dose | Inhibition (%) of capsaicin (10 n mol/kg, iv) response | | | | |
|---|---|---|---|---|---|
| | 2 minutes | 17 minutes | 32 minutes | 47 minutes | 62 minutes |
| 10 mg/kg, iv | 16.6±5.1 | 40.6±12.2 | 51.2±10.4 | 37.2±19.7 | 47.1±16.7 |

(4) Effect of intratrachea administration of WS-9326A on neurokinin A induced bronchoconstriction in guinea-pigs.

In order to test the effect of inhalation of WS-9326A on the bronchoconstriction. WS-9326A was dissolved in DMSO and administered intratrachea. The method was almost same as mentioned above.
As shown in Table 8, WS-9326A was highly potent.

Table 8

| Inhibition of neurokinin A induced bronchoconstriction by intratrachea administration by WS-9326A. | | | | | |
|---|---|---|---|---|---|
| Dose* | Inhibition (%) of neurokinin A** response | | | | |
| | 20 minutes | 35 minutes | 50 minutes | 65 minutes | n |
| 0.03 mg/kg | 32.3 | 28.4 | 35.6 | 35.5 | 4 |
| 0.3 | 50.6 | 42.4 | 44.9 | 42.0 | 4 |
| 3 | 73.4 | 79.4 | 81.7 | 77.7 | 4 |
| ED$_{50}$ mg/kg | 0.23 | 0.29 | 0.19 | 0.24 | |

* : WS-9326A was dissolved in DMSO
** : 1 n mol/kg iv

(5) Acute toxicity

Acute toxicity of WS-9326A was determined in ddY mice (5 weeks old, male) by a single intraperitoneal injection of graded dose of test compound into 5 mice. The LD50 value of WS-9326A was above 250 mg/kg and below 500 mg/kg (500 mg/kg > LD50 > 250 mg/kg).

The pharmaceutical composition of this invention can be used in the form of a pharmaceutical preparation, for example, in solid, semisolid or liquid form, which contains the WS-9326A, WS-9326B, their derivatives or pharmaceutically acceptable salts thereof, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid or liquid form, and in addition auxiliary, stabilizing, thickening and coloring agents and perfumes may be used. The active object compound is included in the pharmaceutical composition in an amount sufficient to produce the desired effect upon the process or condition of diseases.

While the dosage of therapeutically effective amount of the WS-9326A, WS-9326B, their derivatives or pharmaceutically acceptable salts thereof varies from and also depends upon the age and condition of each individual patient to be treated, a daily dose of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The following examples are given for the purpose of illustrating the present invention.

Example 1

Fermentation

An aqueous seed medium (160 ml) containing soluble starch (1%), sucrose (1%), glucose (1%), cotton seed flour (1%), peptone (0.5%), soybean meal (0.5%) and calcium carbonate (0.2%) (pH was adjusted to 7.0 with 6N of sodium hydroxide) was poured into each of twenty 500 ml Erlenmeyer flasks and sterilized at 120°C for 30 minutes.

A loopful of slant culture of Streptomyces violaceoniger No. 9326 was inoculated to each of the media and cultured on a rotary shaker (220 rpm, 5.1 cm throw) at 30°C for 3 days. The resultant seed culture was inoculated to 160 liter of sterile fermentation medium consisting of glycerin (3%), soybean meal (0.5%), ground soybean powder (1.5%), calcium carbonate (0.2%) and sodium iodide (NaI) (0.001%) in 200-liter stainless steel jar-fermentor. The fermentation was carried out at 30°C for 3 days under aeration of 160 liters/minute and agitation of 200 rpm. An amount of WS-9326A in the fermentation broth was quantified by high performance liquid chromatography (HPLC) using Hitachi Model 655 pump. A steel column (4.6 mm inside diameter, 250 mm length) packed with an R-ODS-5 (YMC-packed column) was used at a flow rate of 1.0 ml/minute.

Mobile phase used was a mixture of methanol and water (8:2). The sample for the HPLC assay was prepared as follows; an equal volume of acetone was added to a broth with vigorous stirring and stand for 1 hr and then centrifuged. The $5\mu\ell$ of supernatant was injected to Hitachi Model 655 sample injector.

Isolation and Purification

An equal volume of acetone was added to the culture broth (150 $\ell$) with stirring. The mixture was allowed to stand at room temperature for one hour and then filtered. The filtrate was concentrated to 80 liter under reduced pressure, and was adjusted to pH 7.0 with 1N hydrochloric acid, and then extracted with 80 liter of ethyl acetate. The extract was concentrated to dryness under reduced pressure and applied to a column of silica gel (Kieselgel 60, 70-230 mesh, Merck, 3 $\ell$). The column was washed with n-hexane (10 $\ell$), n-hexane-ethyl acetate [1:1] (10 $\ell$), ethyl acetate (20 $\ell$), and active substance was eluted from the column with acetone (6 $\ell$). The active fractions were dried under reduced pressure, and was subjected to a column chromatography on silica gel (Kieselgel 60, 70-230 mesh, Merck, 1.2 $\ell$). The column was washed with chloroform-methanol [20:1] (5 $\ell$), and the object substance was eluted with a solution of chloroform-methanol [10:1] (6 $\ell$). The fraction was dried under reduced pressure to give a powder. The powder was dissolved in a small volume of methanol and applied to a column of NS gel (Nihon Seimitsu, 500 ml). The object substance was eluted with methanol-water [8:2] (2 ℓ) and concentrated to 300 ml under reduced pressure, and then extracted with 500 ml of ethyl acetate. The extract was concentrated to dryness under reduced pressure to give a powder (5 g). The powder (5 g) was dissolved in 10 ml of methanol (500 mg/ml) and applied to HPLC using a steel column (20 mm inside diameter, 250 mm length) packed with D-ODS-5 (YMC-packed column) and eluted with a mixture of Methanol and water [8:2] at a flow rate of 9.9 ml/minute. Thus obtained active fraction was concentrated under reduced pressure, and then extracted with ethyl acetate. The extract was concentrated to dryness under reduced pressure to give a pure white powder (150 mg) of WS-9326A.

Example 2

To a solution of WS-9326A (300 mg) in pyridine (4.5 ml) were added acetic anhydride (1.5 ml) and 4-dimethylaminopyridine (1 mg) and the reaction mixture was allowed to stand at room temperature overnight. The reaction mixture was evaporated to dryness to afford an oil which was purified by preparative TLC (chloroform-methanol (10:1)).

The obtained product was triturated from diethyl ether to give triacetyl-WS-9326A (332 mg) as a colorless powder. Physical and chemical properties of the triacetyl-WS-9326A are as follows.

(1) Form and Color : colorless powder

(2) Color Reaction :

    Positive :      cerium sulfate reaction, sulfuric acid reaction, iodine vapor reaction

    Negative :    ninhydrine reaction

(3) Solubility :

    Soluble :             methanol, dimethyl sulfoxide

    Sparingly Soluble :    chloroform, diethyl ether

    Insoluble :        n-hexane

(4) Melting Point : 141-143°C

(5) Specific Rotation :

    $[\alpha]_D^{23}$ : -122° (C = 1.0, MeOH)

(6) Ultraviolet Absorption Spectrum :

$$\lambda_{max}^{MeOH} = 283 \text{ nm } (\varepsilon = 32,000)$$

(7) Molecular Formula : $C_{60}H_{74}N_8O_{16}$

(8)

| Elemental Analysis : | | | |
|---|---|---|---|
| Found : | C 60.19, | H 6.42, | N 9.27 |
| Calcd. for $C_{60}H_{74}N_8O_{16} \cdot 2H_2O$ : | C 60.09, | H 6.56, | N 9.34 |

(9) Molecular Weight :

    FAB-MS :    m/z 1163.6 $(M + H)^+$

(10) Thin Layer Chromatography :

| Stationary phase | Developing solvent | Rf value |
|---|---|---|
| Silica gel plate (Merck Art 5715) | Chloroform-methanol (10:1, V/V) | 0.50 |
| | Ethyl acetate | 0.12 |

(11) Infrared Absorption Spectrum :

$$\nu^{KBr}_{max} = 3350,\ 3020,\ 2950,\ 2920,\ 2850,\ 1730,\ 1650,$$
$$1520,\ 1440,\ 1360,\ 1230,\ 1200,\ 1160,\ 1100,$$
$$1060,\ 1040,\ 910\ cm^{-1}$$

(12) Property of the Substance :
neutral substance

(13) $^{13}$C Nuclear Magnetic Resonance Spectrum : (100 MHz, CDCl$_3$-CD$_3$OH (10:1)) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q), |

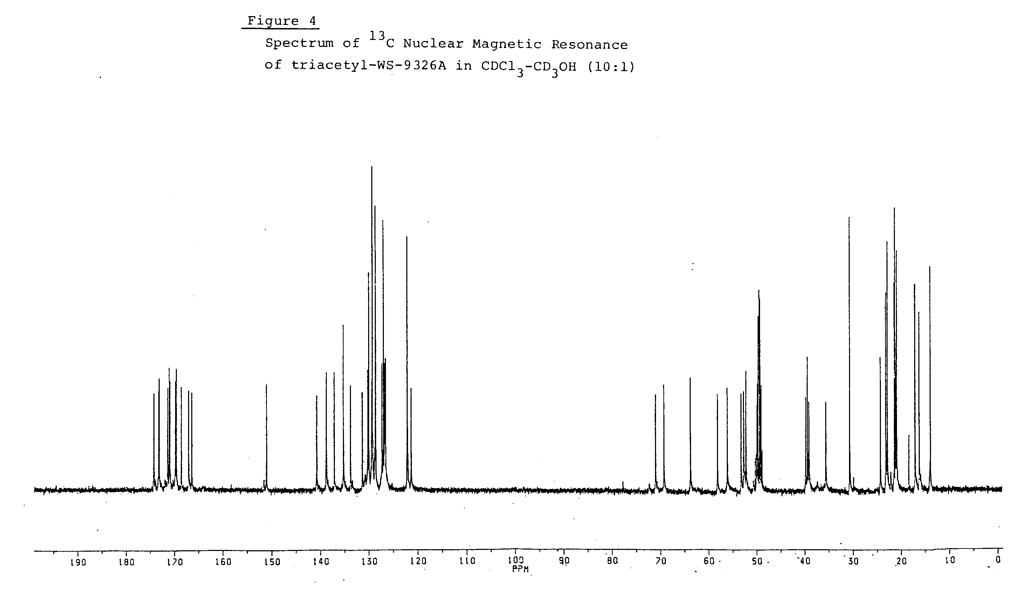

the chart of which is shown in Figure 4,

(14) $^1$H Nuclear Magnetic Resonance Spectrum : (400 MHz, CDCl$_3$CD$_3$OH (10:1)) $\delta$

$$8.25\ (1H,\ d,\ J=8Hz),$$
$$8.02\ (1H,\ d,\ J=8Hz),$$
$$7.88\ (1H,\ d,\ J=16Hz),$$

```
7.86        (1H, d, J=8Hz),
7.70        (1H, d, J=6Hz),
7.61        (1H, d, J=8Hz),
7.45        (1H, d, J=7Hz),
7.32-7.15 (6H, m),
7.03        (2H, d, J=8Hz),
7.00-6.94 (3H, m),
6.88-6.79 (4H, m),
6.70        (1H, s),
6.49        (1H, d, J=12Hz),
5.76        (1H, dt, J=12 and 7.5Hz),
5.54        (1H, broad s),
5.50-5.45 (2H, m),
4.93        (1H, m),
4.75        (1H, m),
4.65-4.56 (2H, m),
4.46        (1H, dd, J=6 and 11Hz),
4.31        (1H, t, J=6Hz),
4.22        (1H, m),
4.18        (1H, dd, J=8 and 11Hz),
3.56        (3H, s),
2.90        (1H, dd, J=6 and 16Hz),
2.85-2.80 (2H, m),
2.56        (1H, dd, J=4 and 16Hz),
2.26        (3H, s),
2.00        (3H, s),
1.96-1.89 (2H, m),
1.85        (3H, s),
1.58        (1H, m),
1.35        (3H, d, J=6Hz),
1.32-1.20 (3H, m),
1.07        (3H, d, J=6Hz),
0.84        (1H, m),
0.72        (3H, d, J=6Hz),
0.71        (3H, t, J=7.5Hz),


0.65          (3H, d, J=6Hz),
```

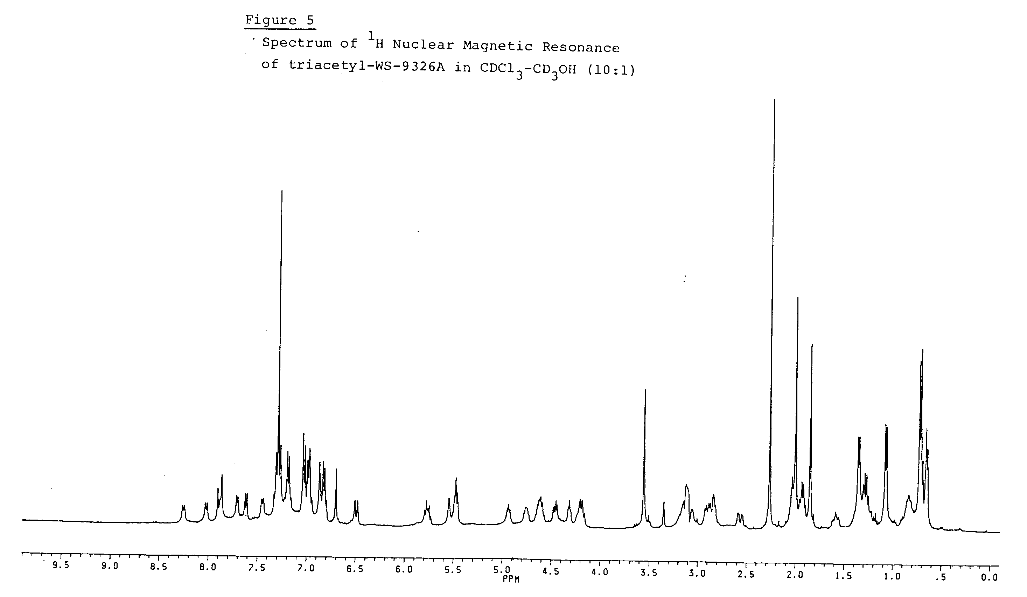

the chart of which is shown in Figure 5.

Example 3

Fermentation

An aqueous seed medium (160 ml) containing soluble starch (1%), sucrose (1%), glucose (1%), cotton seed flour (1%), peptone (0.5%), soybean meal (0.5%) and calcium carbonate (0.2%) was poured into each of ten 500-ml Erlenmeyer flasks and sterilized at 120°C for 30 minutes.

A loopful of slant culture of Streptomyces violaceoniger No. 9326 was inoculated to each of the media and cultured on a rotary shaker (220 rpm, 5.1 cm throw) at 30°C for 3 days.

The resultant seed culture was inoculated to the aqueous seed medium (160 ℓ) containing soluble starch (1%), sucrose (1%), glucose (1%), cotton seed flour (1%), peptone (0.5%), soybean meal (0.5%), calcium carbonate (0.2%), Adekanol LG-109 (defoaming agent, Trademark : Asahi Denka Co.) (0.07%) and Silicone KM-70 (defoaming agent, Trademark : Shin-etsu Chemical Co.) (0.05%) in a 500-liter stainless steel jar-fermentor which had been sterilized at 120°C for 30 minutes in advance. The fermentation was carried out at 30°C for 1 day under aeration of 160 liters/minute and agitation of 200 rpm.

The resultant seed cultured broth (60 ℓ) was inoculated to a sterilized production medium containing glycerin (3.0%), soybean meal (1.0%), chicken meat bone meal (1.0%), calcium carbonate (0.2%), sodium iodide (0.001%), Adekanol LG-109 (0.07%) and Silicone KM-70 (0.05%) in a 4,000-liter stainless steel jar-fermentor which had been sterilized at 120°C for 30 minutes in advance, and cultured at 30°C for 4 days under aeration of 3,000 liters/minute and agitation of 100 rpm.

The progress of the fermentation was monitored by high performance liquid chromatography (HPLC) using Hitachi Model 655 pump. A steel column packed with a reverse phase silica gel "YMC-packed column R-ODS-5" (Trademark, Yamamura Chemical Institute) was used at a flow rate of 1.0 ml/minute. Mobile phase used was an aqueous solution of 45% acetonitrile. The sample for the HPLC assay was prepared as follows; an equal volume of acetone was added to a broth with vigorous stirring and the mixture was stand for one hour and then centrifuged. The 5 $\mu\ell$ of supernatant was injected to the injector of Hitachi Model 655 HPLC.

Isolation and Purification

The cultured broth thus obtained was filtered with an aid of diatomaceous earth (Perlite Topko #34, Trademark, Showa Chemical Industry Co., Ltd.) (15 kg. The mycelial cake was extracted with ethyl acetate (1600 ℓ) and the extract was filtered. The filtrate (1400 ℓ) was applied to a column of active carbon (Sirasagi KL, Trademark, Takeda Pharmaceutical Co., Ltd.) (200 ℓ). The column was washed with ethyl acetate (120 ℓ) and then the elution was carried out with ethyl acetate-methanol [5:1]. The active fractions (fractions from 50 ℓ to 1030 ℓ) were combined and concentrated to 45 ℓ under reduced pressure. n-Hexane (120 ℓ) was added to the resultant solution with stirring. The mixture was allowed to stand at room temperature for one hour and then filtered with an aid of Silika #600 (Chuo Silica Co., Ltd.) (3 kg). The cake thus obtained was washed with n-hexane (15 ℓ) and the object substances were eluted with methanol (20 ℓ).

The eluate was concentrated to dryness under reduced pressure. The residue (500 g) was dissolved with methanol-acetic acid-dichloromethane [1:1:2] (4 ℓ) and applied to a column of silica gel (Kieselgel 60, 70-230 mesh, 70 ℓ) The column was developed with methanol-acetic acid-dichloromethane [1:1:2] (0.5 ℓ) and dichloromethane (25 ℓ). The object substances were eluted with dichloromethane-methanol [10:1] and dichloromethane-methanol [8:1]. The active fractions were combined and concentrated under reduced pressure. The residue was dissolved with methanol (1 ℓ). Acetonitrile (9 ℓ) was added to the resultant solution with stirring. The mixture was allowed to stand at room temperature for one hour and the resultant precipitate was collected by filtration. This precipitation step was repeated three times. The precipitate thus obtained was washed with acetonitrile (1 ℓ) and dried to give a white powder (190 g) of WS-9326A. The filtrates thus obtained from these precipitation steps were combined and concentrated to dryness under reduced pressure. The residue (11.7 g) was dissolved with 80% aqueous methanol and resultant solution was passed through a column of active carbon (300 ml). The column was washed with 80% aqueous methanol (1 ℓ) and the elution was carried out with methanol (6 ℓ). The active fractions were combined and concentrated to dryness under reduced pressure. The residue (3.4 g) was dissolved with methanol (12 ml). The resultant solution was applied to a column of reverse phase silica gel (YMC packed column R-354 S-15/30 (ODS), $\phi$50 x ℓ 300 mm x 2; maker, Yamamura Chemical Institute) equilibrated with 50% aqueous acetonitrile. The column was developed with 50% aqueous acetonitrile using Waters HPLC (System 500). The eluates containing WS-9326B (fractions from 3 ℓ to 3.5 ℓ) were combined and concentrated to dryness to give a white powder (790 mg) of WS-9326 B.

Example 4

To a solution of WS-9326A (100 mg) in pyridine (1 ml) was added acetic anhydride (0.01 ml) and the mixture was allowed to stand at room temperature overnight. The mixture was evaporated to dryness to afford an oil which was purified by preparative TLC (chloroform-methanol (9:1)). The obtained product was triturated with diethyl ether to give monoacetyl-WS-9326A (55 mg) as a colorless powder. Physical and chemical properties of the monoacetyl-WS-9326A are as follows.

(1) Form and Color : colorless powder
(2) Molecular Formula : $C_{56}H_{70}N_8O_{14}$
(3) Molecular Weight :
   FAB-MS :    m/z 1079.4 $(M+H)^+$
(4) Thin Layer Chromatography :

| Stationary phase | Developing solvent | Rf value |
|---|---|---|
| Silica gel plate Merck Art 5715) | Chloroform-methanol (10:1, V/V) | 0.17 |

(5) Infrared Absorption Spectrum :

$$\nu_{max}^{KBr} = 3300, 2920, 1730, 1650, 1500,$$
$$1360, 1190, 1170, 910 \text{ cm}^{-1}$$

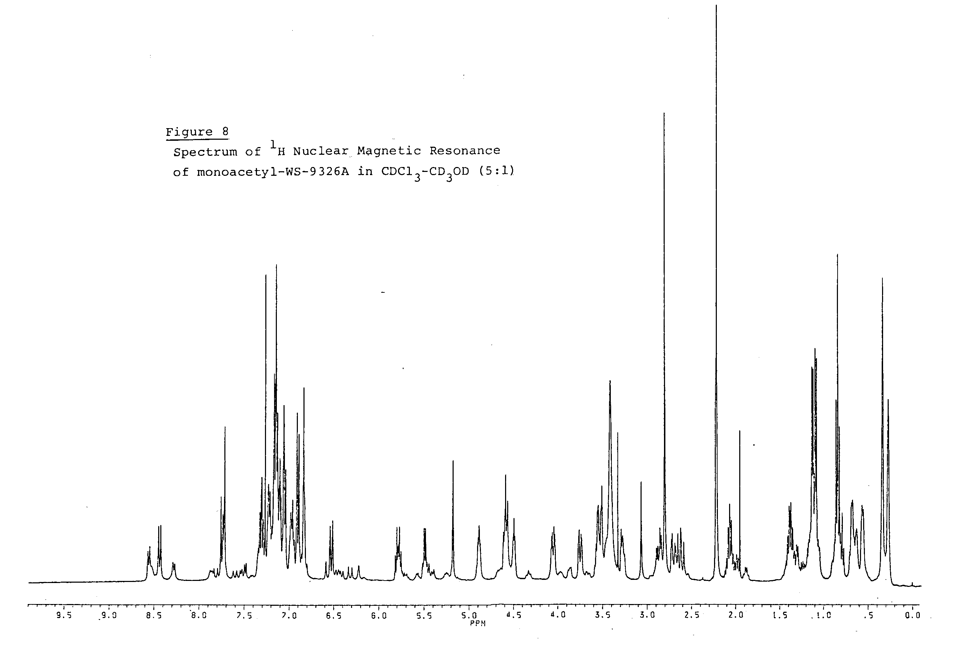

(6) Property of the Substance :
   neutral substance
(7) $^1$H Nuclear Magnetic Resonance Spectrum (400 MHz, $CDC\ell_3$-$CD_3OD$ (5:1)) :
   the chart of which is shown in Figure 8.

Example 5

To a solution of WS-9326A (100 mg) in pyridine (1 ml) was added acetic anhydride (0.03 ml) and the mixture was allowed to stand at room temperature overnight. The mixture was evaporated to dryness to afford an oil which was purified by preparative TLC (chloroform-methanol (9:1)) to give diacetyl-WS-9326A (72 mg) as a colorless powder. Physical and chemical properties of the diacetyl-WS-9326A are as follows.

(1) Form and color : colorless powder
(2) Molecular Formula : $C_{58}H_{72}N_8O_{15}$
(3) Molecular Weight :
   FAB-MS :    m/z 1121.4 $(M+H)^+$
(4) Thin Layer Chromatography :

| Stationary phase | Developing solvent | Rf value |
|---|---|---|
| Silica gel plate (Merck Art 5715) | Chloroform-methanol (10:1 V/V) | 0.35 |

(5) Infrared Absorption Spectrum :

$$\nu_{max}^{KBr} = 3300, 3020, 2950, 1730, 1650, 1520, 1500,$$
$$1360, 1200, 1170, 1100, 1040, 980, 910$$
$$\text{cm}^{-1}$$

22

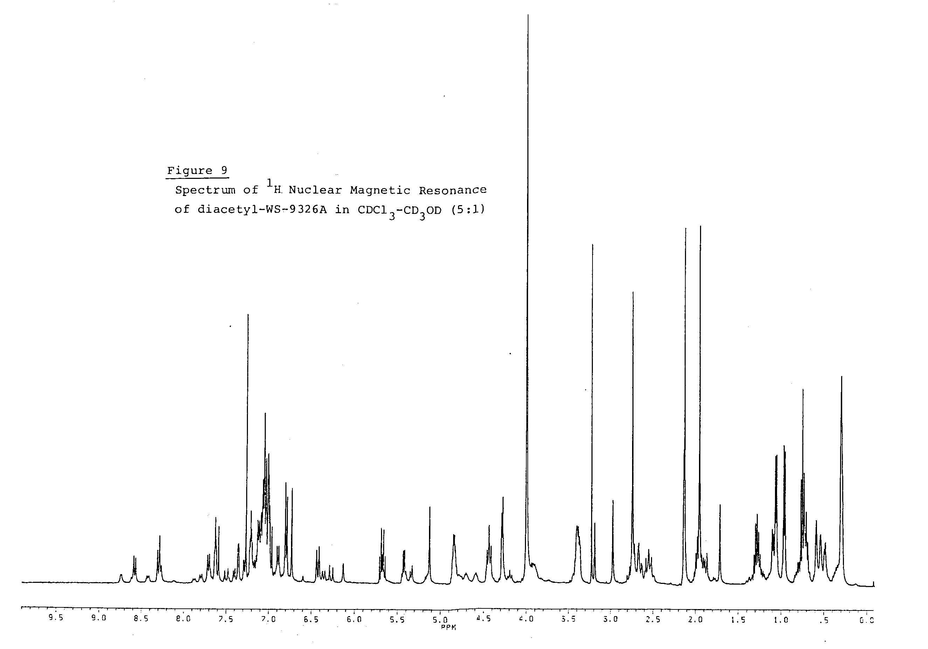

(6) Property of the Substance :
   neutral substance
(7) $^1$H Nuclear Magnetic Resonance Spectrum (400 MHz, CDC$\ell_3$-CD$_3$OD (5:1)) :
the chart of which is shown in Figure 9.

**Claims**
**Claims for the following Contracting States : AT, BE, CH, DE, FR, GB, IT, LI, LU, NL, SE**

1.  A compound selected from WS-9326A, WS-9326B, WS-9326A substituted with 1 to 3 acyl group(s) and WS-9326B substituted with 1 to 3 acyl group(s), and a pharmaceutically acceptable salt thereof, wherein

(i) WS-9326A has the following physical and chemical properties :
   (1) Form and Color :
      Colorless powder
   (2) Color Reaction :
      Positive :     cerium sulfate reaction, iodine vapor reaction, ferric chloride-potassium ferricyanide reaction,
      Negative :     ninhydrine reaction, Molish reaction, ferric chloride reaction, Ehrlich reaction, Pauli reaction
   (3) Solubility :
      Soluble :                methanol, ethanol
      Sparingly Soluble :     acetone, ethyl acetate
      Insoluble :               water, chloroform
   (4) Melting Point : 187-190 °C
   (5) Specific Rotation :
      $[\alpha]_D^{23}$ : -84° (C = 1.0 MeOH)
   (6) Ultraviolet Absorption Spectrum :

$$\lambda_{max}^{MeOH} = 280 \ nm \ (\varepsilon=34,700)$$

   (7) Infrared Absorption Spectrum :

$$\nu_{Max}^{KBr} = 3300, \ 3050, \ 2950, \ 2920, \ 2860, \ 1730,$$
$$1650, \ 1610, \ 1560, \ 1540, \ 1530, \ 1510,$$
$$1440, \ 1380, \ 1340, \ 1280, \ 1240, \ 1170,$$
$$1110, \ 1080, \ 1060, \ 1040, \ 970, \ 920,$$
$$880, \ 860, \ 830 \ cm^{-1}$$

   (8)

| Elementary Analysis : | | | |
|---|---|---|---|
| Found : | C 60.18, | H 6.61, | N 10.32 |
| Calcd. for $C_{54}H_{68}N_8O_{13} \cdot 2H_2O$ : | C 60.43, | H 6.76, | N 10.44 |

   (9) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.46 |

(10) Molecular Formula : $C_{54}H_{68}N_8O_{13}$

(11) Molecular Weight :

FAB-MS :      m/z 1037 $(M+H)^+$

(12) Property of the Substance :

acidic substance

(13) $^{13}C$ Nuclear Magnetic Resonance Spectrum : (100 MHz, $CD_3OD$) $\delta$

| | |
|---|---|
| 175.69 (s), | 174.70 (s), |
| 173.73 (s), | 173.38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1H$ Nuclear Magnetic Resonance Spectrum : (400 MHz, $CD_3OD$) $\delta$

| | |
|---|---|
| 7.80 | (1H, d, J = 8Hz), |
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 and 7Hz), |
| 5.55 | (1H, m), |
| 5.35 | (1H, broad signal), |
| 5.10 | (1H, dd, J = 3 and 9.5Hz), |
| 4.68 | (1H, d, J = 10Hz), |
| 4.55 | (1H, t, J = 6Hz), |
| 4.48 | (1H, dd, J = 3 and 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 and 14Hz), |
| 2.94 | (1H, dd, J = 3 and 16Hz), |
| 2.89 | (3H, s), |
| 2.74 | (1H, dd, J = 9.5 and 16Hz), |
| 2.69 | (1H, dd, J = 12 and 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B has the following physical and chemical properties :

(1) Form and Color : colorless amorphous powder

(2) Color Reaction :

Positive :      cerium sulfate reaction, iodine vapor reaction

Negative :      ninhydrine reaction

(3) Solubility :

Soluble :              methanol

Sparingly Soluble :    ethanol

Insoluble :            water, acetone, ethyl acetate, chloroform

(4) Melting Point : 165-170 °C (dec.)

(5) Specific Rotation : $[\alpha]_D^{23}$ : -64 ° (C = 1.0 MeOH)

(6) Ultraviolet Absorption Spectrum :

$$\lambda_{max}^{MeOH} = 283 \ nm \ (\varepsilon = 27,000)$$

(7) Molecular Formula : $C_{54}H_{70}N_8O_{13}$

(8)

| Elemental Analysis : | | | |
|---|---|---|---|
| Found : | C 59.97, | H 6.87, | N 10.29 |
| Calcd. for $C_{54}H_{70}N_8O_{13} \cdot 2H_2O$ : | C 60.32, | H 6.94, | N 10.42 |

(9) Molecular Weight :

    FAB-MS :      m/z 1061.6 $(M + Na)^+$

(10) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.25 |

(11) Infrared Absorption Spectrum :

$$\nu_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) $^{13}C$ Nuclear Magnetic Resonance Spectrum : (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Nuclear Magnetic Resonance Spectrum : (400 MHz, $CD_3OD$) $\delta$

| 7.86 | (1H, d, J = 16Hz), |
|---|---|
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 and 11.5Hz), |
| 5.08 | (1H, dd, J = 3.5 and 10Hz), |
| 5.04 | (1H, q, J = 6.5Hz), |
| 4.66 | (1H, dd, J = 3.5 and 13Hz), |
| 4.65 | (1H, d, J = 11.5Hz), |
| 4.56 | (1H, dd, J = 2.5 and 7Hz), |
| 4.48 | (1H, dd, J = 4.5 and 11Hz), |
| 4.46 | (1H, s), |
| 3.88 | (2H, m), |
| 3.64 | (2H, m), |
| 3.51 | (1H, dd, J = 3.5 and 14Hz), |
| 3.17 | (1H, dd, J = 4.5 and 14Hz), |
| 3.01 | (1H, dd, J = 11 and 14Hz), |
| 2.94 | (1H, dd, J = 3.5 and 16Hz), |
| 2.71 | (3H, s), |
| 2.71 | (1H, dd, J = 10 and 16Hz), |
| 2.64 | (1H, dd, J = 13 and 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

2. A compound of claim 1,
wherein acyl group is lower alkanoyl.

3. A compound of claim 2,
which is triacetyl-WS-9326A having the following physical and chemical properties :
(1) Form and Color : colorless powder
(2) Color Reaction :
    Positive :     cerium sulfate reaction, sulfuric acid reaction, iodine vapor reaction
    Negative :     ninhydrine reaction
(3) Solubility :
    Soluble :     methanol, dimethyl sulfoxide
    Sparingly Soluble :     chloroform, diethyl ether
    Insoluble :     n-hexane
(4) Melting Point : 141-143 °C
(5) Specific Rotation : $[\alpha]_D^{23}$ : -122° (C = 1.0, MeOH)
(6) Ultraviolet Absorption Spectrum :

$$\lambda_{max}^{MeOH} = 283 \ nm \ (\varepsilon = 32,000)$$

(7) Molecular Formula : $C_{60}H_{74}N_8O_{16}$
(8)

27

| Elemental Analysis : | | | |
|---|---|---|---|
| Found : | C 60.19, | H 6.42, | N 9.27 |
| Calcd. for $C_{60}H_{74}N_8O_{16} \cdot 2H_2O$ : | C 60.09, | H 6.56, | N 9.34 |

(9) Molecular Weight :

FAB-MS :     m/z 1163.6 $(M+H)^+$

(10) Thin Layer Chromatography :

| Stationary phase | Developing solvent | Rf value |
|---|---|---|
| Silica gel plate (Merck Art 5715) | chloroform-methanol (10:1, V/V) ethyl acetate | 0.50 0.12 |

(11) Infrared Absorption Spectrum :

$$\nu_{max}^{KBr} = 3350, 3020, 2950, 2920, 2850, 1730,$$
$$1650, 1520, 1440, 1360, 1230, 1200,$$
$$1160, 1100, 1060, 1040, 910 \ cm^{-1}$$

(12) Property of the Substance :

neutral substance

(13) $^{13}C$ Nuclear Magnetic Resonance Spectrum : (100 MHz, $CDC\ell_3$-$CD_3OH$ (10:1)) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (g), |
| 20.83 (q), | 17.05 (q), |
| 16.18 (q), | 13.82 (q) |

(14)H[1] Nuclear Magnetic Resonance Spectrum : (400 MHz, CDC$\ell_3$-CD$_3$OH (10:1)) $\delta$

```
8.25        (1H, d, J=8Hz),
8.02        (1H, d, J=8Hz),
7.88        (1H, d, J=16Hz),
7.86        (1H, d, J=8Hz),
7.70        (1H, d, J=6Hz),
7.61        (1H, d, J=8Hz),
7.45        (1H, d, J=7Hz),
7.32-7.15 (6H, m),
7.03        (2H, d, J=8Hz),
7.00-6.94 (3H, m),
6.88-6.79 (4H, m),
6.70        (1H, s),

6.49        (1H, d, J=12Hz),
5.76        (1H, dt, J=12 and 7.5Hz),
5.54        (1H, broad s),
5.50-5.45 (2H, m),
4.93        (1H, m),
4.75        (1H, m)
4.65-4.56 (2H, m),
4.46        (1H, dd, J=6 and 11Hz),
4.31        (1H, t, J=6Hz),
4.22        (1H, m),
4.18        (1H, dd, J=8 and 11Hz),
3.56        (3H, s),
2.90        (1H, dd, J=6 and 16Hz),
2.85-2.80 (2H, m),
2.56        (1H, dd, J=4 and 16Hz),
2.26        (3H, s),
2.00        (3H, s),
1.96-1.89 (2H, m),
1.85        (3H, s),
1.58        (1H, m),
1.35        (3H, d, J=6Hz),
1.32-1.20 (3H, m),
1.07        (3H, d, J=6Hz),
0.84        (1H, m),
0.72        (3H, d, J=6Hz),
0.71        (3H, t, J=7.5Hz),
0.65        (3H, d, J=6Hz)
```

4.  A process for the production of the compound selected from WS-9326A and WS-9326B according to claim 1, or a salt thereof, which comprises culturing a WS-9326A and/or WS-9326B producing strain belonging to the genus <u>Streptomyces</u> in nutrient medium and recovering a compound selected from WS-9326A and WS-9326B, or a salt thereof from the resultant cultured broth.

5. A process for the production of a compound selected from WS-9326A substituted with 1 to 3 acyl group(s) and WS-9326B substituted with 1 to 3 acyl group(s) according to claim 1, or a salt thereof, which comprises reacting a compound selected from WS-9326A and WS-9326B, or a salt thereof with acylating agent(s).

6. A pharmaceutical composition containing a compound selected from WS-9326A, WS-9326B, WS-9326A substituted with 1 to 3 acyl groups(s) and WS-9326B substituted with 1 to 3 acyl groupl(s) according to claim 1, or a pharmaceutically acceptable salt thereof, as an active ingredient, in association with a pharmaceutically acceptable, substantially non-toxic carrier or excipient.

7. A use of a compound selected from WS-9326A, WS-9326B, WS-9326A substituted with 1 to 3 acyl group(s) and WS-9326B substituted with 1 to 3 acyl group(s) according to claim 1, or a pharmaceutically acceptable salt thereof for the treatment and prevention of asthme.

8. A biologically pure culture of the microorganism <u>Streptomyces</u> <u>violaceoniger</u> No. 9326 (FERM BP-1667).

**Claims for the following Contracting State : ES**

1. A process for the production of
a compound selected from WS-9326A, WS-9326B, WS-9326A substituted with 1 to 3 acyl group(s) and WS-9326B substituted with 1 to 3 acyl group(s), and a salt thereof wherein
(i) WS-9326A has the following physical and chemical properties :
(1) Form and Color :
Colorless powder
(2) Color Reaction :
Positive :     cerium sulfate reaction, iodine vapor reaction, ferric chloride-potassium ferricyanide reaction,
Negative :     ninhydrine reaction, Molish reaction, ferric chloride reaction, Ehrlich reaction, Pauli reaction
(3) Solubility :
Soluble :               methanol, ethanol
Sparingly Soluble :     acetone, ethyl acetate
Insoluble :             water, chloroform
(4) Melting Point : 187-190°C
(5) Specific Rotation :
$[\alpha]_D^{23}$ : -84° (C = 1.0, MeOH)
(6) Ultraviolet Absorption Spectrum :

$$\lambda_{max}^{MeOH} = 280 \text{ nm } (\varepsilon = 34,700)$$

(7) Infrared Absorption Spectrum :

$$\nu^{KBr}_{Max} = 3300, 3050, 2950, 2920, 2860, 1730,$$
$$1650, 1610, 1560, 1540, 1530, 1510,$$
$$1440, 1380, 1340, 1280, 1240, 1170,$$
$$1110, 1080, 1060, 1040, 970, 920,$$
$$880, 860, 830 \ cm^{-1}$$

(8)

| Elementary Analysis : | | | |
|---|---|---|---|
| Found : | C 60.18, | H 6.61, | N 10.32 |
| Calcd. for $C_{54}H_{68}N_8O_{13} \cdot 2H_2O$ : | C 60.43, | H 6.76, | N 10.44 |

(9) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.46 |

(10) Molecular Formula : $C_{54}H_{68}N_8O_{13}$
(11) Molecular Weight :
   FAB-MS :    m/z 1037 $(M+H)^+$
(12) Property of the Substance :
   acidic substance
(13) $^{13}C$ Nuclear Magnetic Resonance Spectrum : (100 MHz, $CD_3OD$) $\delta$

| | |
|---|---|
| 175.69 (s), | 174.70 (s), |
| 173.73 (s), | 173.38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1$H Nuclear Magnetic Resonance Spectrum : (400 MHz, $CD_3OD$) $\delta$

| | |
|---|---|
| 7.80 | (1H, d, J = 8Hz), |
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 and 7Hz), |
| 5.55 | (1H, m), |
| 5.35 | (1H, broad signal), |
| 5.10 | (1H, dd, J = 3 and 9.5Hz), |
| 4.68 | (1H, d, J = 10Hz), |
| 4.55 | (1H, t, J = 6Hz), |
| 4.48 | (1H, dd, J = 3 and 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 and 14Hz), |
| 2.94 | (1H, dd, J = 3 and 16Hz), |
| 2.89 | (3H, s), |
| 2.74 | (1H, dd, J = 9.5 and 16Hz), |
| 2.69 | (1H, dd, J = 12 and 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B has the following physical and chemical properties :

(1) Form and Color : colorless amorphous powder

(2) Color Reaction :

Positive : cerium sulfate reaction, iodine vapor reaction

Negative : ninhydrine reaction

(3) Solubility :

Soluble : methanol

Sparingly Soluble : ethanol

Insoluble : water, acetone, ethyl acetate, chloroform

(4) Melting Point : 165-170 °C (dec.)

(5) Specific Rotation : $[\alpha]_D^{23}$ : -64° (C = 1.0, MeOH)

(6) Ultraviolet Absorption Spectrum :

$$\lambda_{max}^{MeOH} = 283 \ nm \ (\varepsilon = 27,000)$$

(7) Molecular Formula : $C_{54}H_{70}N_8O_{13}$

(8)

| Elemental Analysis : | | | |
|---|---|---|---|
| Found : | C 59.97, | H 6.87, | N 10.29 |
| Calcd. for $C_{54}H_{70}N_8O_{13} \cdot 2H_2O$ : | C 60.32, | H 6.94, | N 10.42 |

(9) Molecular Weight :

    FAB-MS :     m/z 1061.6 $(M+Na)^+$

(10) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.25 |

(11) Infrared Absorption Spectrum :

$$\nu_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) $^{13}C$ Nuclear Magnetic Resonance Spectrum : (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Nuclear Magnetic Resonance Spectrum : (400 MHz, $CD_3OD$) $\delta$

35

| | |
|---|---|
| 7.86 | (1H, d, J = 16Hz), |
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 and 11.5Hz), |
| 5.08 | (1H, dd, J = 3.5 and 10Hz), |
| 5.04 | (1H, q, J = 6.5Hz), |
| 4.66 | (1H, dd, J = 3.5 and 13Hz), |
| 4.65 | (1H, d, J = 11.5Hz), |
| 4.56 | (1H, dd, J = 2.5 and 7Hz), |
| 4.48 | (1H, dd, J = 4.5 and 11Hz), |
| 4.46 | (1H, s), |
| 3.88 | (2H, m), |
| 3.64 | (2H, m), |
| 3.51 | (1H, dd, J = 3.5 and 14Hz), |
| 3.17 | (1H, dd, J = 4.5 and 14Hz), |
| 3.01 | (1H, dd, J = 11 and 14Hz), |
| 2.94 | (1H, dd, J = 3.5 and 16Hz), |
| 2.71 | (3H, s), |
| 2.71 | (1H, dd, J = 10 and 16Hz), |
| 2.64 | (1H, dd, J = 13 and 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

which comprises culturing a WS-9326A and/or WS-9326B producing strain belonging to the genus Streptomyces in nutrient medium and recovering a compound selected from WS-9326A and WS-9326B, or a salt thereof from the resultant cultured broth.

2. The process of claim 1 for the production of a compound selected
from WS-9326A substituted with 1 to 3 acyl group(s) and WS-9326B substituted with 1 to 3 acyl group-(s), or a salt thereof,
which comprises reacting a compound selected from WS-9326A and WS-9326B, or a salt thereof with acylating agent(s).

3. The process of claim 1 for the production of WS-9326A or a salt thereof.

4. The process of claim 1 for the production of WS-9326B or a salt thereof.

5. The process of claim 1 for the production of WS-9326A substituted with 1 to 3 acyl group(s) or a or a salt thereof.

6. The process of claim 5 wherein acyl group is lower alkanoyl.

7. The process of claim 6 for the production of triacetyl-WS-9326A having the following physical and chemical properties:
   (1) Form and Color : colorless powder
   (2) Color Reaction :
       Positive :    cerium sulfate reaction, sulfuric acid reaction, iodine vapor reaction
       Negative :    ninhydrine reaction

36

(3) Solubility :

    Soluble :                 methanol, dimethyl sulfoxide

    Sparingly Soluble :     chloroform, diethyl ether

    Insoluble :           n-hexane

(4) Melting Point : 141-143 °C

(5) Specific Rotation : $[\alpha]_D^{23}$ : -122 ° (C = 1.0, MeOH)

(6) Ultraviolet Absorption Spectrum :

$$\lambda_{max}^{MeOH} = 283 \text{ nm } (\varepsilon = 32{,}000)$$

(7) Molecular Formula : $C_{60}H_{74}N_8O_{16}$

(8)

| Elemental Analysis : | | | |
|---|---|---|---|
| Found : | C 60.19, | H 6.42, | N 9.27 |
| Calcd. for $C_{60}H_{74}N_8O_{16} \cdot 2H_2O$ : | C 60.09, | H 6.56, | N 9.34 |

(9) Molecular Weight :

    FAB-MS :     m/z 1163.6 $(M + H)^+$

(10) Thin Layer Chromatography :

| Stationary phase | Developing solvent | Rf value |
|---|---|---|
| Silica gel plate (Merck Art 5715) | chloroform-methanol (10:1, V/V) | 0.50 |
| | ethyl acetate | 0.12 |

(11) Infrared Absorption Spectrum :

$$\nu_{max}^{KBr} = 3350, 3020, 2950, 2920, 2850, 1730,$$
$$1650, 1520, 1440, 1360, 1230, 1200,$$
$$1160, 1100, 1060, 1040, 910 \text{ cm}^{-1}$$

(12) Property of the Substance :

    neutral substance

(13) $^{13}C$ Nuclear Magnetic Resonance Spectrum : (100 MHz, $CDC\ell_3$-$CD_3OH$ (10:1)) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (g), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q) |

(14)H[1] Nuclear Magnetic Resonance Spectrum : (400 MHz, CDC$\ell_3$-CD$_3$OH (10:1)) δ

```
8.25        (1H, d, J=8Hz),
8.02        (1H, d, J=8Hz),
7.88        (1H, d, J=16Hz),
7.86        (1H, d, J=8Hz),
7.70        (1H, d, J=6Hz),
7.61        (1H, d, J=8Hz),
7.45        (1H, d, J=7Hz),
7.32-7.15 (6H, m),
7.03        (2H, d, J=8Hz),
7.00-6.94 (3H, m),
6.88-6.79 (4H, m),
6.70        (1H, s),
6.49        (1H, d, J=12Hz),
5.76        (1H, dt, J=12 and 7.5Hz),
5.54        (1H, broad s),
5.50-5.45 (2H, m),
4.93        (1H, m),
4.75        (1H, m)
4.65-4.56 (2H, m),
4.46        (1H, dd, J=6 and 11Hz),
4.31        (1H, t, J=6Hz),
4.22        (1H, m),
4.18        (1H, dd, J=8 and 11Hz),
3.56        (3H, s),
2.90        (1H, dd, J=6 and 16Hz),
2.85-2.80 (2H, m),
2.56        (1H, dd, J=4 and 16Hz),
2.26        (3H, s),
2.00        (3H, s),
1.96-1.89 (2H, m),
1.85        (3H, s),
1.58        (1H, m),
1.35        (3H, d, J=6Hz),
1.32-1.20 (3H, m),
1.07        (3H, d, J=6Hz),
0.84        (1H, m),
0.72        (3H, d, J=6Hz),
0.71        (3H, t, J=7.5Hz),
0.65        (3H, d, J=6Hz)
```

EP 0 327 009 B1

**Claims for the following Contracting State : GR**

1. A process for the production of a compound selected from WS-9326A, WS-9326B, WS-9326A substituted with 1 to 3 acyl group(s) and WS-9326B substituted with 1 to 3 acyl group(s), and a salt thereof wherein

    (i) WS-9326A has the following physical and chemical properties :

        (1) Form and Color :

            Colorless powder

        (2) Color Reaction :

            Positive :      cerium sulfate reaction, iodine vapor reaction, ferric chloride-potassium ferricyanide reaction,

            Negative :    ninhydrine reaction, Molish reaction, ferric chloride reaction, Ehrlich reaction, Pauli reaction

        (3) Solubility :

            soluble :             methanol, ethanol

            Sparingly Soluble :    acetone, ethyl acetate

            Insoluble :         water, chloroform

        (4) Melting Point : 187-190 °C

        (5) Specific Rotation :

            $[\alpha]_D^{23}$ : -84° (C = 1.0, MeOH)

        (6) Ultraviolet Absorption Spectrum :

$$\lambda_{max}^{MeOH} = 280 \ nm \ (\varepsilon = 34,700)$$

        (7) Infrared Absorption Spectrum :

$$\nu_{Max}^{KBr} = 3300, \ 3050, \ 2950, \ 2920, \ 2860, \ 1730,$$
$$1650, \ 1610, \ 1560, \ 1540, \ 1530, \ 1510,$$
$$1440, \ 1380, \ 1340, \ 1280, \ 1240, \ 1170,$$
$$1110, \ 1080, \ 1060, \ 1040, \ 970, \ 920,$$
$$880, \ 860, \ 830 \ cm^{-1}$$

        (8)

| Elementary Analysis : | | | |
|---|---|---|---|
| Found : | C 60.18, | H 6.61, | N 10.32 |
| Calcd. for $C_{54}H_{68}N_8O_{13} \cdot 2H_2O$ : | C 60.43, | H 6.76, | N 10.44 |

        (9) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.46 |

        (10) Molecular Formula : $C_{54}H_{68}N_8O_{13}$

40

(11) Molecular Weight :

    FAB-MS :     m/z 1037 $(M+H)^+$

(12) Property of the Substance :

    acidic substance

(13) $^{13}C$ Nuclear Magnetic Resonance Spectrum : (100 MHz, $CD_3OD$) $\delta$

| | |
|---|---|
| 175.69 (s), | 174.70 (s), |
| 173.73 (s), | 173.38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1H$ Nuclear Magnetic Resonance Spectrum : (400 MHz, $CD_3OD$) $\delta$

| 7.80 | (1H, d, J = 8Hz), |
|---|---|
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 and 7Hz), |
| 5.55 | (1H, m), |
| 5.35 | (1H, broad signal), |
| 5.10 | (1H, dd, J = 3 and 9.5Hz), |
| 4.68 | (1H, d, J = 10Hz), |
| 4.55 | (1H, t, J = 6Hz), |
| 4.48 | (1H, dd, J = 3 and 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 and 14Hz), |
| 2.94 | (1H, dd, J = 3 and 16Hz), |
| 2.89 | (3H, s), |
| 2.74 | (1H, dd, J = 9.5 and 16Hz), |
| 2.69 | (1H, dd, J = 12 and 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B has the following physical and chemical properties :

(1) Form and Color : colorless amorphous powder

(2) Color Reaction :

Positive : cerium sulfate reaction, iodine vapor reaction

Negative : ninhydrine reaction

(3) Solubility :

Soluble : methanol

Sparingly Soluble : ethanol

Insoluble : water, acetone, ethyl acetate, chloroform

(4) Melting Point : 165-170 °C (dec.)

(5) Specific Rotation : $[\alpha]_D^{23}$ : -64 ° (C = 1.0, MeOH)

(6) Ultraviolet Absorption Spectrum :

$$\lambda_{max}^{MeOH} = 283 \ nm \ (\varepsilon = 27,000)$$

(7) Molecular Formula : $C_{54}H_{70}N_8O_{13}$

(8)

| Elemental Analysis : | | | |
|---|---|---|---|
| Found : | C 59.97, | H 6.87, | N 10.29 |
| Calcd. for $C_{54}H_{70}N_8O_{13} \cdot 2H_2O$ : | C 60.32, | H 6.94, | N 10.42 |

(9) Molecular Weight :

    FAB-MS :     m/z 1061.6 $(M + Na)^+$

(10) Thin Layer Chromatography :

| Stationary phase | Developing solvent | Rf value |
|---|---|---|
| Silica gel plate (Merck Art 5715) | chloroform-methanol (5:1, V/V) | 0.38 |
| RP-18 plate | methanol-water (8:2, V/V) | 0.25 |

(11) Infrared Absorption Spectrum :

$$\nu_{max}^{KBr} = 3300,\ 3050,\ 2950,\ 1735,\ 1660,\ 1530,$$
$$1510,\ 1450,\ 1400,\ 1380,\ 1340,\ 1260,$$
$$1220,\ 1080,\ 980,\ 920\ cm^{-1}$$

(12) $^{13}C$ Nuclear Magnetic Resonance Spectrum : (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Nuclear Magnetic Resonance Spectrum : (400 MHz, $CD_3OD$) $\delta$

| | |
|---|---|
| 7.86 | (1H, d, J = 16Hz), |
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 and 11.5Hz), |
| 5.08 | (1H, dd, J = 3.5 and 10Hz), |
| 5.04 | (1H, q, J = 6.5Hz), |
| 4.66 | (1H, dd, J = 3.5 and 13Hz), |
| 4.65 | (1H, d, J = 11.5Hz), |
| 4.56 | (1H, dd, J = 2.5 and 7Hz), |
| 4.48 | (1H, dd, J = 4.5 and 11Hz), |
| 4.46 | (1H, s), |
| 3.88 | (2H, m), |
| 3.64 | (2H, m), |
| 3.51 | (1H, dd, J = 3.5 and 14Hz), |
| 3.17 | (1H, dd, J = 4.5 and 14Hz), |
| 3.01 | (1H, dd, J = 11 and 14Hz), |
| 2.94 | (1H, dd, J = 3.5 and 16Hz), |
| 2.71 | (3H, s), |
| 2.71 | (1H, dd, J = 10 and 16Hz), |
| 2.64 | (1H, dd, J = 13 and 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

which comprises culturing a WS-9326A and/or WS-9326B producing strain belonging to the genus Streptomyces in nutrient medium and recovering a compound selected from WS-9326A and WS-9326B, or a salt thereof from the resultant cultured broth.

2. The process of claim 1 for the production of a compound selected from WS-9326A substituted with 1 to 3 acyl group(s) and WS-9326B substituted with 1 to 3 acyl group-(s), or a salt thereof, which comprises reacting a compound selected from WS-9326A and WS-9326B, or a salt thereof with acylating agent(s).

3. The process of claim 1 for the production of WS-9326A or a salt thereof.

4. The process of claim 1 for the production of WS-9326B or a salt thereof.

5. The process of claim 1 for the production of WS-9326A substituted with 1 to 3 acyl group(s) or a or a salt thereof.

6. The process of claim 5 wherein acyl group is lower alkanoyl.

7. The process of claim 6 for the production of triacetyl-WS-9326A having the following physical and chemical properties:
   (1) Form and Color : colorless powder
   (2) Color Reaction :
      Positive :    cerium sulfate reaction, sulfuric acid reaction, iodine vapor reaction
      Negative :    ninhydrine reaction

44

(3) Solubility :

    Soluble :                methanol, dimethyl sulfoxide

    Sparingly Soluble :    chloroform, diethyl ether

    Insoluble :           n-hexane

(4) Melting Point : 141-143 °C

(5) Specific Rotation : $[\alpha]_D^{23}$ : -122° (C = 1.0, MeOH)

(6) Ultraviolet Absorption Spectrum :

$$\lambda_{max}^{MeOH} = 283 \text{ nm } (\varepsilon = 32,000)$$

(7) Molecular Formula : $C_{60}H_{74}N_8O_{16}$

(8)

| Elemental Analysis : | | | |
|---|---|---|---|
| Found : | C 60.19, | H 6.42, | N 9.27 |
| Calcd. for $C_{60}H_{74}N_8O_{16} \cdot 2H_2O$ : | C 60.09, | H 6.56, | N 9.34 |

(9) Molecular Weight :

    FAB-MS :    m/z 1163.6 $(M + H)^+$

(10) Thin Layer Chromatography :

| Stationary phase | Developing solvent | Rf value |
|---|---|---|
| Silica gel plate (Merck Art 5715) | chloroform-methanol (10:1, V/V) | 0.50 |
| | ethyl acetate | 0.12 |

(11) Infrared Absorption Spectrum :

$$\nu_{max}^{KBr} = 3350, \ 3020, \ 2950, \ 2920, \ 2850, \ 1730,$$
$$1650, \ 1520, \ 1440, \ 1360, \ 1230, \ 1200,$$
$$1160, \ 1100, \ 1060, \ 1040, \ 910 \text{ cm}^{-1}$$

(12) Property of the Substance :

    neutral substance

(13) $^{13}$C Nuclear Magnetic Resonance Spectrum : (100 MHz, $CDC\ell_3$-$CD_3OH$ (10:1)) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q) |

(14)H[1] Nuclear Magnetic Resonance Spectrum : (400 MHz CDC$\ell_3$-CD$_3$OH (10:1)) δ

```
8.25       (1H, d, J=8Hz),
8.02       (1H, d, J=8Hz),
7.88       (1H, d, J=16Hz),
7.86       (1H, d, J=8Hz),
7.70       (1H, d, J=6Hz),
7.61       (1H, d, J=8Hz),
7.45       (1H, d, J=7Hz),
7.32-7.15 (6H, m),
7.03       (2H, d, J=8Hz),
7.00-6.94 (3H, m),
6.88-6.79 (4H, m),
6.70       (1H, s),
6.49       (1H, d, J=12Hz),
5.76       (1H, dt, J=12 and 7.5Hz),
5.54       (1H, broad s),
5.50-5.45 (2H, m),
4.93       (1H, m),
4.75       (1H, m)
4.65-4.56 (2H, m),
4.46       (1H, dd, J=6 and 11Hz),
4.31       (1H, t, J=6Hz),
4.22       (1H, m),
4.18       (1H, dd, J=8 and 11Hz),
3.56       (3H, s),
2.90       (1H, dd, J=6 and 16Hz),
2.85-2.80 (2H, m),
2.56       (1H, dd, J=4 and 16Hz),
2.26       (3H, s),
2.00       (3H, s),
1.96-1.89 (2H, m),
1.85       (3H, s),
1.58       (1H, m),
1.35       (3H, d, J=6Hz),
1.32-1.20 (3H, m),
1.07       (3H, d, J=6Hz),
0.84       (1H, m),
0.72       (3H, d, J=6Hz),
0.71       (3H, t, J=7.5Hz),
0.65       (3H, d, J=6Hz)
```

8. Modification of the process of any of claims 1 to 7, which is characterized by bringing a compound prepared by the process of any of claims 1 to 7 or a pharmaceutically acceptable salt thereof into pharmaceutically acceptable form by admixture or presentation of said compound with a pharmaceutically acceptable diluent or carrier.

9. A biologically pure culture of the microorganism *Streptomyces* *violaceoniger* No. 9326 (FERM BP-1667).

**Patentansprüche**
**Patentansprüche für folgende Vertragsstaaten : AT, BE, CH, DE, FR, GB, IT, LI, LU, NL, SE**

1. Verbindung ausgewählt aus: WS-9326A, WS-9326B, WS-9326A substituiert mit 1 bis 3 Acylgruppe(n) und WS-9326B substituiert mit 1 bis 3 Acylgruppe(n), und ein pharmazeutisch verträgliches Salz davon, worin

    (i) WS-9326A die folgenden physikalischen und chemischen Eigenschaften aufweist:

        (1) Form und Farbe: Farbloses Pulver

        (2) Farbreaktion:

            Positiv:     Cersulfat-Reaktion, Joddampf-Reaktion, Eisenchlorid-Kaliumhexacyanoferrat (III)-Reaktion

            Negativ:     Ninhydrin-Reaktion, Molisch-Reaktion, Eisenchlorid-Reaktion, Ehrlich-Reaktion, Pauli-Reaktion

        (3) Löslichkeit:

            Löslich:         Methanol, Ethanol

            Mäßig löslich:    Aceton, Ethylacetat

            Unlöslich:       Wasser, Chloroform

        (4) Schmelzpunkt: 187-190 °C

        (5) Spezifische Drehung: $[\alpha]^{23}_D$ : -84 ° (C = 1,0; MeOH)

        (6) Ultraviolettes Absorptionsspektrum:

$$\lambda^{MeOH}_{max} = 280 \text{ nm } (\xi = 34700)$$

        (7) Infrarotes Absorptionsspektrum:

$$\nu^{KBr}_{max} = 3300, 3050, 2950, 2920, 2860, 1730,$$
$$1650, 1610, 1560, 1540, 1530, 1510,$$
$$1440, 1380, 1340, 1280, 1240, 1170,$$
$$1110, 1080, 1060, 1040, 970, 920,$$
$$880, 860, 830 \text{ cm}^{-1}$$

        (8)

| Elementaranalyse: | | | |
|---|---|---|---|
| Gef.: | C 60,18; | H 6,61; | N 10,32 |
| Ber. für $C_{54}H_{68}N_8O_{13} \cdot 2H_2O$ : | C 60,43; | H 6,76; | N 10,44 |

        (9) Dünnschichtchromatographie:

| Stationäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,46 |

(10) Molekularformel: $C_{54}H_{68}N_8O_{13}$

(11) Molekulargewicht: FAB-MS : m/z 1037 (M + H)$^+$

(12) Eigenschaft der Substanz: Saure Substanz

(13) $^{13}$C Magnetisches Kernresonanzspektrum: (100 MHz, CD$_3$OD) δ

| | |
|---|---|
| 175,69 (s), | 174,70 (s), |
| 173,73 (s), | 173,38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (9), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1$H Magnetisches Kernresonanzspektrum: (400 MHz, CD$_3$OD) δ

| | |
|---|---|
| 7.80 | (1H, d, J = 8Hz), |
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 und 7Hz), |
| 5.55 | (1H, m), |
| 5.35 | (1H, breites Signal), |
| 5.10 | (1H, dd, J = 3 und 9.5Hz), |
| 4.68 | (1H, d, J = 10Hz), |
| 4.55 | (1H, t, J = 6Hz), |
| 4.48 | (1H, dd, J = 3 und 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 und 14Hz), |
| 2.94 | (1H, dd, J = 3 und 16Hz), |
| 2.89 | (3H, s), |
| 2.74 | (1H, dd, J = 9.5 und 16Hz), |
| 2.69 | (1H, dd, J = 12 und 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B hat die folgenden physikalischen und chemischen Eigenschaften:

(1) Form und Farbe: Farbloses amorphes Pulver

(2) Farbreaktion:

Positiv: Cersulfat-Reaktion, Joddampf-Reaktion

Negativ: Ninhydrin-Reaktion

(3) Löslichkeit:

Löslich: Methanol

Mäßig löslich: Ethanol

Unlöslich: Wasser, Aceton, Ethylacetat, Chloroform

(4) Schmelzpunkt: 165-170 °C (Zers.)

(5) Spezifische Drehung: $[\alpha]^{23}_D$ : -64 ° (C = 1,0; MeOH)

(6) Ultraviolettes Absorptionsspektrum:

$$\lambda^{MeOH}_{max} = 283 \text{ nm } (\varepsilon = 27000)$$

(7) Molekularformel:

$C_{54}H_{70}N_8O_{13}$

(8)

| Elementaranalyse: | | | |
|---|---|---|---|
| Gef.: | C 59,97; | H 6,87; | N 10,29 |
| Ber. für $C_{54}N_{70}N_8O_{13} \cdot 2H_2O$ : | C 60,32; | H 6,94; | N 10,42 |

(9) Molekulargewicht: FAB-MS: m/z 1061,6 $(M+Na)^+$

(10) Dünnschichtchromatographie:

| Stätionäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,25 |

(11) Infrarotes Absorptionspektrum:

$$\gamma_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) $^{13}C$ Magnetisches Kernresonanzspektrum: (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Magnetisches Kernresonanzspektrum: (400 MHz, $CD_3OD$) $\delta$

| 7.86 | (1H, d, J = 16Hz), |
|---|---|
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 und 11.5Hz), |
| 5.08 | (1H, dd, J = 3.5 und 10Hz), |
| 5.04 | (1H, q, J = 6.5Hz), |
| 4.66 | (1H, dd, J = 3.5 und 13Hz), |
| 4.65 | (1H, d, J = 11.5Hz), |
| 4.56 | (1H, dd, J = 2.5 und 7Hz), |
| 4.48 | (1H, dd, J = 4.5 und 11Hz), |
| 4.46 | (1H, s), |
| 3.88 | (2H, m), |
| 3.64 | (2H, m), |
| 3.51 | (1H, dd, J = 3.5 und 14Hz), |
| 3.17 | (1H, dd, J = 4.5 und 14Hz), |
| 3.01 | (1H, dd, J = 11 und 14Hz), |
| 2.94 | (1H, dd, J = 3.5 und 16Hz), |
| 2.71 | (3H, s), |
| 2.71 | (1H, dd, J = 10 und 16Hz), |
| 2.64 | (1H, dd, J = 13 und 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

2. Verbindung nach Anspruch 1, worin die Acylgruppe nieder Alkanoyl ist.

3. Verbindung nach Anspruch 2, die Triacetyl-WS-9326A ist, das die folgenden physikalischen und chemischen Eigenschaften besitzt:

(1) Form und Farbe: Farbloses Pulver

(2) Farbreaktion:

Positiv: Cersulfat-Reaktion, Schwefelsäure-Reaktion, Joddampf-Reaktion

Negativ: Ninhydrin-Reaktion

(3) Löslichkeit:

Löslich: Methanol, Dimethylsulfoxid

Mäßig löslich: Chloroform, Diethylether

Unlöslich: n-Hexan

(4) Schmelzpunkt: 141-143 °C

(5) Spezifische Drehung: $[\alpha]^{23}_D$ : -122 ° (C = 1,0; MeOH)

(6) Ultraviolettes Absorptionsspektrum:

$$\lambda^{MeOH}_{max} = 283 \text{ nm } (\mathcal{E} = 32000)$$

(7) Molekularformel: $C_{60}H_{74}N_8O_{16}$

(8)

52

EP 0 327 009 B1

| Elementaranalyse: | | | |
|---|---|---|---|
| Gef.: | C 60,19; | H 6,42; | N 9,27 |
| Ber. für $C_{60}H_{74}N_8O_{16} \cdot 2H_2O$: | C 60,09; | H 6,56; | N 9,34 |

(9) Molekulargewicht: FAB-MS: m/z 1163,6 $(M+H)^+$

(10) Dünnschichtchromatographie:

| Stationäre Phase | Entwicklungslösungsmittel | Rf Wert |
|---|---|---|
| Silicagel-Platte (Merck Art. 5715) | Chloroform-Methanol (10:1, V/V)<br>Ethylacetat | 0,50<br>0,12 |

(11) Infrarotes Absorptionsspektrum:

$$\gamma_{max}^{KBr} = 3350, 3020, 2950, 2920, 2850, 1730,$$
$$1650, 1520, 1440, 1360, 1230, 1200,$$
$$1160, 1100, 1060, 1040, 910 \text{ cm}^{-1}$$

(12) Eigenschaft der Substanz: Neutrale Substanz

(13) $^{13}$C Magnetisches Kernresonanzspektrum: (100 MHz, $CDCl_3$-$CD_3OH$ (10:1)) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q) |

(14) $^1$H Magnetisches Kernresonanzspektrum: (400 MHz, $CDCl_3$-$CD_3OH$ (10:1)) $\delta$

53

| | |
|---|---|
| 8.25 | (1H, d, J = 8Hz), |
| 8.02 | (1H, d, J = 8Hz), |
| 7.88 | (1H, d, J = 16Hz), |
| 7.86 | (1H, d, J = 8Hz), |
| 7.70 | (1H, d, J = 6Hz), |
| 7.61 | (1H, d, J = 8Hz), |
| 7.45 | (1H, d, J = 7Hz), |
| 7.32-7.15 | (6H, m), |
| 7.03 | (2H, d, J = 8Hz), |
| 7.00-6.94 | (3H, m), |
| 6.88-6.79 | (4H, m), |
| 6.70 | (1H, s), |
| 6.49 | (1H, d, J = 12Hz), |
| 5.76 | (1H, dt, J = 12 und 7.5Hz), |
| 5.54 | (1H, breit s), |
| 5.50-5.45 | (2H, m), |
| 4.93 | (1H, m), |
| 4.75 | (1H, m) |
| 4.65-4.56 | (2H, m), |
| 4.46 | (1H, dd, J = 6 und 11Hz), |
| 4.31 | (1H, t, J = 6Hz), |
| 4.22 | (1H, m), |
| 4.18 | (1H, dd, J = 8 und 11Hz), |
| 3.56 | (3H, s), |
| 2.90 | (1H, dd, J = 6 und 16Hz), |
| 2.85-2.80 | (2H, m), |
| 2.56 | (1H, dd, J = 4 und 16Hz), |
| 2.26 | (3H, s), |
| 2.00 | (3H, s), |
| 1.96-1.89 | (2H, m), |
| 1.85 | (3H, s), |
| 1.58 | (1H, m), |
| 1.35 | (3H, d, J = 6Hz), |
| 1.32-1.20 | (3H, m), |
| 1.07 | (3H, d, J = 6Hz), |
| 0.84 | (1H, m), |
| 0.72 | (3H, d, J = 6Hz), |
| 0.71 | (3H, t, J = 7.5Hz), |
| 0.65 | (3H, d, J = 6Hz) |

4. Verfahren zur Herstellung einer Verbindung, ausgewählt aus WS-9326A und WS-9326B, gemäß Anspruch 1, oder eines Salzes davon, welches das Kultivieren eines WS-9326A- und/oder WS-9326B-produzierendenStammes, der zur Gattung Streptomyces gehört, in einem Nährstoffmedium und das Gewinnen einer Verbindung, ausgewählt aus WS-9326A und WS-9326B, oder eines Salzes davon, aus der resultierenden Nährlösung umfaßt.

5. Verfahren zur Herstellung einer Verbindung, ausgewählt aus WS-9326A substituiert mit 1 bis 3 Acylgruppe(n) und WS-9326B substituiert mit 1 bis 3 Acylgruppe(n), gemäß Anspruch 1, oder eines Salzes davon, welches das Reagieren einer Verbindung, ausgewählt aus WS-9326A und WS-9326B, oder eines Salzes davon, mit acylierenden Mittel(n) umfaßt.

6. Pharmazeutische Zusammensetzung, die eine Verbindung, ausgewählt aus WS-9326A, WS-9326B, WS-9326A substituiert mit 1 bis 3 Acylgruppe(n) und WS-9326B substituiert mit 1 bis 3 Acylgruppe(n), gemäß Anspruch 1, oder ein pharmazeutisch verträgliches Salz davon als aktiven Bestandteil in Assoziation mit einem pharmazeutisch verträglichen, im wesentlichen nichttoxischen Träger oder Exzipienten enthält.

**7.** Verwendung einer Verbindung, ausgewählt aus WS-9326A, WS-9326B, WS-9326A substituiert mit 1 bis 3 Acylgruppe(n) und WS-9326B substituiert mit 1 bis 3 Acylgruppe(n), gemäß Anspruch 1, oder eines pharmazeutisch verträgliches Salz davon zur Behandlung und Prävention von Asthma.

**8.** Biologisch reine Kultur des Mikroorganismus <u>Streptomyces</u> <u>violaceoniger</u> Nr. 9326 (FERM BP-1667).

**Patentansprüche für folgenden Vertragsstaat : ES**

**1.** Verfahren zur Herstellung einer Verbindung ausgewählt aus: WS-9326A, WS-9326B, WS-9326A substituiert mit 1 bis 3 Acylgruppe(n) und WS-9326B substituiert mit 1 bis 3 Acylgruppe(n), und eines Salzes davon, worin

  (i) WS-9326A die folgenden physikalischen und chemischen Eigenschaften aufweist:

    (1) Form und Farbe:

      Farbloses Pulver

    (2) Farbreaktion:

      Positiv:    Cersulfat-Reaktion, Joddampf-Reaktion, Eisenchlorid-Kaliumhexacyanoferrat(III)-Reaktion

      Negativ:   Ninhydrin-Reaktion, Molisch-Reaktion, Eisenchlorid-Reaktion, Ehrlich-Reaktion, Pauli-Reaktion

    (3) Löslichkeit:

      Löslich:      Methanol, Ethanol

      Mäßig löslich:  Aceton, Ethylacetat

      Unlöslich:   Wasser, Chloroform

    (4) Schmelzpunkt: 187-190 °C

    (5) Spezifische Drehung: $[\alpha]^{23}_D$ : -84° (C = 1,0; MeOH)

    (6) Ultraviolettes Absorptionsspektrum:

$$\lambda^{MeOH}_{max} = 280 \text{ nm } (\mathcal{E} = 34700)$$

    (7) Infrarotes Absorptionsspektrum:

$$\nu^{KBr}_{max} = 3300, 3050, 2950, 2920, 2860, 1730,$$
$$1650, 1610, 1560, 1540, 1530, 1510,$$
$$1440, 1380, 1340, 1280, 1240, 1170,$$
$$1110, 1080, 1060, 1040, 970, 920,$$
$$880, 860, 830 \text{ cm}^{-1}$$

    (8)

| Elementaranalyse: | | | |
|---|---|---|---|
| Gef.: | C 60,18; | H 6,61; | N 10,32 |
| Ber. für $C_{54}H_{68}N_8O_{13} \cdot 2H_2O$: | C 60,43; | H 6,76; | N 10,44 |

    (9) Dünnschichtchromatographie:

| Stationäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,46 |

(10) Molekularformel: $C_{54}H_{68}N_8O_{13}$

(11) Molekulargewicht: FAB-MS: m/z 1037 $(M+H)^+$

(12) Eigenschaft der Substanz: Saure Substanz

(13) $^{13}C$ Magnetisches Kernresonanzspektrum: (100 MHz, $CD_3OD$) δ

| | |
|---|---|
| 175,69 (s), | 174,70 (s), |
| 173,73 (s), | 173,38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d), | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1H$ Magnetisches Kernresonanzspektrum: (400 MHz, $CD_3OD$) δ

| | |
|---|---|
| 7.80 | (1H, d, J = 8Hz), |
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 und 7Hz), |
| 5.55 | (1H, m), |
| 5.35 | (1H, breites Signal) |
| 5.10 | (1H, dd, J = 3 und 9.5Hz), |
| 4.68 | (1H, d, J = 10Hz), |
| 4.55 | (1H, t, J = 6Hz), |
| 4.48 | (1H, dd, J = 3 und 12Hz), |
| 3.92 | (2H, d, J = 6Hz), |
| 3.70 | (1H, t, J = 7.5Hz), |
| 3.62 | (1H, m), |
| 3.46 | (1H, dd, J = 3 und 14Hz), |
| 2.94 | (1H, dd, J = 3 und 16Hz), |
| 2.89 | (3H, s), |
| 2.74 | (1H, dd, J = 9.5 und 16Hz), |
| 2.69 | (1H, dd, J = 12 und 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B die folgenden physikalischen und chemischen Eigenschaften aufweist:

(1) Form und Farbe: Farbloses amorphes Pulver

(2) Farbreaktion:

Positiv: Cersulfat-Reaktion, Joddampf-Reaktion

Negativ: Ninhydrin-Reaktion

(3) Löslichkeit:

Löslich: Methanol

Mäßig löslich: Ethanol

Unlöslich: Wasser, Aceton, Ethylacetat, Chloroform

(4) Schmelzpunkt: 165-170 °C (Zers.)

(5) Spezifische Drehung: $[\alpha]^{23}_D$ : -64 ° (C = 1,0; MeOH)

(6) Ultraviolettes Absorptionsspektrum:

$$\lambda^{MeOH}_{max} = 283 \ nm \ (\mathcal{E} = 27000)$$

(7) Molekularformel: $C_{54}H_{70}N_8O_{13}$

(8)

| Elementaranalyse: | | | |
|---|---|---|---|
| Gef.: | C 59,97; | H 6,87; | N 10,29 |
| Ber. für $C_{54}H_{70}N_8O_{13} \cdot 2H_2O$ : | C 60,32; | H 6,94; | N 10,42 |

(9) Molekulargewicht: FAB-MS m/z 1061,6 $(M + Na)^+$
(10) Dilnnschichtchromatographie:

| Stätionäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,25 |

(11) Infrarotes Absorptionspektrum:

$$\nu_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) $^{13}C$ Magnetisches Kernresonanzspektrum: (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Magnetisches Kernresonanzspektrum: (400 MHz, $CD_3OD$) $\delta$

58

| 7.86 | (1H, d, J = 16Hz), |
|---|---|
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 und 11.5Hz), |
| 5.08 | (1H, dd, J = 3.5 und 10Hz), |
| 5.04 | (1H, q, J = 6.5Hz), |
| 4.66 | (1H, dd, J = 3.5 und 13Hz), |
| 4.65 | (1H, d, J = 11.5Hz), |
| 4.56 | (1H, dd, J = 2.5 und 7Hz), |
| 4.48 | (1H, dd, J = 4.5 und 11Hz), |
| 4.46 | (1H, s), |
| 3.88 | (2H, m), |
| 3.64 | (2H, m), |
| 3.51 | (1H, dd, J = 3.5 und 14Hz), |
| 3.17 | (1H, dd, J = 4.5 und 14Hz), |
| 3.01 | (1H, dd, J = 11 und 14Hz), |
| 2.94 | (1H, dd, J = 3.5 und 16Hz), |
| 2.71 | (3H, s), |
| 2.71 | (1H, dd, J = 10 und 16Hz), |
| 2.64 | (1H, dd, J = 13 und 14Hz), |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

welches das Kultivieren eines WS-9326A- und/oder WS-9326B-produzierenden Stammes, der zur Gattung Streptomyces gehört, in einem Nährstoffmedium und das Gewinnen einer Verbindung, ausgewählt aus WS-9326A und WS-9326B, oder eines Salzes davon, aus der resultierenden Nährlösung umfaßt.

2. Verfahren nach Anspruch 1 zur Herstellung einer Verbindung, ausgewählt aus: WS-9326A substituiert mit 1 bis 3 Acylgruppe(n), WS-9326B substituiert mit 1 bis 3 Acylgruppe(n), oder eines Salzes davon, welches das Reagieren einer Verbindung, ausgewählt aus WS-9326A und WS-9326B, oder eines Salzes davon, mit acylierendem(n) Mittel(n) umfaßt.

3. Verfahren nach Anspruch 1 zur Herstellung von WS-9326A oder eines Salzes davon.

4. Verfahren nach Anspruch 1 zur Herstellung von WS-9326B oder eines Salzes davon.

5. Verfahren nach Anspruch 1 zur Herstellung von WS-9326A substituiert mit 1 bis 3 Acylgruppe(n) oder eines Salzes davon.

6. Verfahren nach Anspruch 5, worin die Acylgruppe nieder Alkanoyl ist.

7. Verfahren nach Anspruch 6 zur Herstellung von Triacetyl-WS-9326A mit folgenden physikalischen und chemischen Eigenschaften:
   (1) Form und Farbe: Farbloses Pulver
   (2) Farbreaktion:
       Positiv:    Cersulfat-Reaktion, Schwefelsäure-Reaktion, Joddampf-Reaktion
       Negativ:    Ninhydrin-Reaktion

(3) Löslichkeit:

Löslich: Methanol, Dimethylsulfoxid
Mäßig löslich: Chloroform, Diethylether
Unlöslich: n-Hexan

(4) Schmelzpunkt: 141-143 °C

(5) Spezifische Drehung: $[\alpha]^{23}_D$ : -122° (C = 1,0; MeOH)

(6) Ultraviolettes Absorptionsspektrum:

$$\lambda^{MeOH}_{max} = 283 \text{ nm } (\varepsilon = 32000)$$

(7) Molekularformel: $C_{60}H_{74}N_8O_{16}$

(8)

| Elementaranalyse: | | | |
|---|---|---|---|
| Gef.: | C 60,19; | H 6,42; | N 9,27 |
| Ber. für $C_{60}H_{74}N_8O_{16} \cdot 2H_2O$: | C 60,09; | H 6,56; | N 9,34 |

(9) Molekulargewicht: FAB-MS: m/z 1163,6 $(M + H)^+$

(10) Dünnschichtchromatographie:

| Stationäre Phase | Entwicklungslösungsmittel | Rf Wert |
|---|---|---|
| Silicagel-Platte (Merck Art. 5715) | Chloroform-Methanol (10:1, V/V) | 0,50 |
| | Ethylacetat | 0,12 |

(11) Infrarotes Absorptionsspektrum:

$$\nu^{KBr}_{max} = 3350, 3020, 2950, 2920, 2850, 1730,$$
$$1650, 1520, 1440, 1360, 1230, 1200,$$
$$1160, 1100, 1060, 1040, 910 \text{ cm}^{-1}$$

(12) Eigenschaft der Substanz: Neutrale Substanz

(13) $^{13}$C Magnetisches Kernresonanzspektrum: (100 MHz, $CDCl_3$-$CD_3OH$ (10:1) ) δ

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q) |

(14) $^1$H Magnetisches Kernresonanzspektrum: (400 MHz, $CDCl_3$-$CD_3OH$ (10:1)) $\delta$

```
8.25        (1H, d, J=8Hz),
8.02        (1H, d, J=8Hz),
7.88        (1H, d, J=16Hz),
7.86        (1H, d, J=8Hz),
7.70        (1H, d, J=6Hz),
7.61        (1H, d, J=8Hz),
7.45        (1H, d, J=7Hz),
7.32-7.15 (6H, m),
7.03        (2H, d, J=8Hz),
7.00-6.94 (3H, m),

6.88-6.79 (4H, m),
6.70        (1H, s),
6.49        (1H, d, J=12Hz),
5.76        (1H, dt, J=12 und 7.5Hz),
5.54        (1H, breit s),
5.50-5.45 (2H, m),
4.93        (1H, m),
4.75        (1H, m)
4.65-4.56 (2H, m),
4.46        (1H, dd, J=6 und 11Hz),
4.31        (1H, t, J=6Hz),
4.22        (1H, m),
4.18        (1H, dd, J=8 und 11Hz),
3.56        (3H, s),
2.90        (1H, dd, J=6 und 16Hz),
2.85-2.80 (2H, m),
2.56        (1H, dd, J=4 und 16Hz),
2.26        (3H, s),
2.00        (3H, s),
1.96-1.89 (2H, m),
1.85        (3H, s),
1.58        (1H, m),
1.35        (3H, d, J=6Hz),
1.32-1.20 (3H, m),
1.07        (3H, d, J=6Hz),
0.84        (1H, m),
0.72        (3H, d, J=6Hz),
0.71        (3H, t, J=7.5Hz),
0.65        (3H, d, J=6Hz)
```

**Patentansprüche für folgenden Vertragsstaat : GR**

1. Verfahren zur Herstellung einer Verbindung ausgewählt aus: WS-9326A, WS-9326B, WS-9326A substituiert mit 1 bis 3 Acylgruppe(n) und WS-9326B substituiert mit 1 bis 3 Acylgruppe(n), und eines Salzes davon, worin

(i) WS-9326A die folgenden physikalischen und chemischen Eigenschaften aufweist:

(1) Form und Farbe:
   Farbloses Pulver

(2) Farbreaktion:
   Positiv:       Cersulfat-Reaktion,   Joddampf-Reaktion,   Eisenchlorid-Kaliumhexacyanoferrat(III)-Reaktion
   Negativ:       Ninhydrin-Reaktion,   Molisch-Reaktion,   Eisenchlorid-Reaktion,   Ehrlich-Reaktion, Pauli-Reaktion

(3) Löslichkeit:
   Löslich:           Methanol, Ethanol
   Mäßig löslich:     Aceton, Ethylacetat
   Unlöslich:         Wasser, Chloroform

(4) Schmelzpunkt: 187-190 °C

(5) Spezifische Drehung: $[\alpha]^{23}_D$ : -84 ° (C = 1,0; MeOH)

(6) Ultraviolettes Absorptionsspektrum:

$$\lambda^{MeOH}_{max} = 280 \text{ nm } (\xi = 34700)$$

(7) Infrarotes Absorptionsspektrum:

$$\gamma^{KBr}_{max} = 3300, \ 3050, \ 2950, \ 2920, \ 2860, \ 1730,$$
$$1650, \ 1610, \ 1560, \ 1540, \ 1530, \ 1510,$$
$$1440, \ 1380, \ 1340, \ 1280, \ 1240, \ 1170,$$
$$1110, \ 1080, \ 1060, \ 1040, \ 970, \ 920,$$
$$880, \ 860, \ 830 \text{ cm}^{-1}$$

(8)

| Elementaranalyse: | | | |
|---|---|---|---|
| Gef.: | C 60,18; | H 6,61; | N 10,32 |
| Ber. für $C_{54}H_{68}N_8O_{13} \cdot 2H_2O$ : | C 60,43; | H 6,76; | N 10,44 |

(9) Dünnschichtchromatographie:

| Stationäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,46 |

(10) Molekularformel: $C_{54}H_{68}N_8O_{13}$

(11) Molekulargewicht: FAB-MS : m/z 1037 (M + H)$^+$

(12) Eigenschaft der Substanz: saure Substanz

(13) $^{13}C$ Magnetisches Kernresonanzspektrum: (100 MHz, CD$_3$OD) δ

| | |
|---|---|
| 175,69 (s), | 174,70 (s), |
| 173,73 (s), | 173,38 (s), |
| 172.89 (s), | 171.04 (s), |
| 170.45 (s), | 167.79 (s), |
| 167.15 (s), | 159.20 (s), |
| 140.05 (d), | 139.12 (s), |
| 138.71 (s), | 135.27 (d), |
| 134.85 (s), | 132.11 (d), |
| 132.03 (s), | 131.69 (d) x 2, |
| 130.70 (d), | 129.90 (d), |
| 129.61 (d) x 2, | 129.22 (d) x 2, |
| 128.55 (d) , | 128.04 (d), |
| 127.99 (d), | 127.38 (d), |
| 126.09 (s), | 123.70 (d), |
| 115.63 (d) x 2, | 73.46 (d), |
| 71.34 (d), | 62.80 (t), |
| 59.53 (d), | 56.91 (d), |
| 56.76 (d), | 55.55 (d), |
| 53.64 (d), | 52.10 (d), |
| 39.85 (t), | 37.18 (t), |
| 37.09 (t), | 34.58 (q), |
| 31.37 (t), | 24.56 (d), |
| 23.63 (t), | 22.71 (q), |
| 22.52 (q), | 21.17 (q), |
| 17.19 (q), | 14.13 (q) |

(14) $^1$H Magnetisches Kernresonanzspektrum: (400 MHz, $CD_3OD$) $\delta$

| 7.80 | (1H, d, J = 8Hz), |
|---|---|
| 7.67 | (1H, d, J = 16Hz), |
| 7.45-7.14 | (9H, m), |
| 7.06 | (2H, d, J = 8Hz), |
| 6.83 | (1H, s), |
| 6.65 | (2H, d, J = 8Hz), |
| 6.59 | (1H, d, J = 12Hz), |
| 5.88 | (1H, dt, J = 12 und 7Hz), |
| 5.55 | (1H, m), |
| 5.35 | (1H, broad signal), |
| 5.10 | (1H, dd, J = 3 und 9.5Hz), |
| 4.68 | (1H, d, J = 10Hz), |
| 4.55 | (1H, t, J = 6Hz), |
| 4.48 | (1H, dd, J = 3 und 12Hz), |
| 3.92 | (2H, d, J = 6HZ), |
| 3.70 | (1H, t, J = 7.5Hz), |
| 3.62 | (1H, m), |
| 3.46 | (1H, dd, J = 3 und 14Hz), |
| 2.94 | (1H, dd, J = 3 und 16Hz), |
| 2.89 | (3H, s), |
| 2.74 | (1H, dd, J = 9.5 und 16Hz), |
| 2.69 | (1H, dd, J = 12 und 14Hz), |
| 2.14 | (2H, m), |
| 1.5-1.4 | (2H, m), |
| 1.20 | (3H, d, J = 6Hz) |
| 1.08 | (3H, d, J = 6Hz), |
| 1.0-0.8 | (2H, m), |
| 0.91 | (3H, t, J = 7Hz), |
| 0.6 | (1H, m) |
| 0.53 | (3H, d, J = 6Hz), |
| 0.51 | (3H, d, J = 6Hz) |

(ii) WS-9326B die folgenden physikalischen und chemischen Eigenschaften aufweist:

(1) Form und Farbe: Farbloses amorphes Pulver

(2) Farbreaktion:
Positiv: Cersulfat-Reaktion, Joddampf-Reaktion
Negativ: Ninhydrin-Reaktion

(3) Löslichkeit:
Löslich: Methanol
Mäßig löslich: Ethanol
Unlöslich: Wasser, Aceton, Ethylacetat, Chloroform

(4) Schmelzpunkt: 165-170 °C (Zers.)

(5) Spezifische Drehung: $[\alpha]^{23}_D$ : -64° (C = 1,0; MeOH)

(6) Ultraviolettes Absorptionsspektrum:

$$\lambda^{MeOH}_{max} = 283 \text{ nm } (\epsilon = 27000)$$

(7) Molekularformel: $C_{54}H_{70}N_8O_{13}$

(8)

| Elementaranalyse: | | | |
|---|---|---|---|
| Gef.: | C 59,97; | H 6,87; | N 10,29 |
| Ber. für $C_{54}H_{70}N_8O_{13} \cdot 2H_2O$ : | C 60,32; | H 6,94; | N 10,42 |

(9) Molekulargewicht: FAB-MS m/z 1061,6 $(M + Na)^+$

(10) Dünnschichtchromatographie:

| Stätionäre Phase | Entwicklungslösungsmittel | Rf Wert |
|---|---|---|
| Silicagel-Platte (Merck Art. 5715) | Chloroform-Methanol (5:1, V/V) | 0,38 |
| RP-18-Platte | Methanol-Wasser (8:2, V/V) | 0,25 |

(11) Infrarotes Absorptionspektrum:

$$\gamma_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) $^{13}c$ Magnetisches Kernresonanzspektrum: (100 MHz, $CD_3OD$) $\delta$

| | |
|---|---|
| 174.99 (s), | 174.54 (s), |
| 173.60 (s), | 173.41 (s), |
| 173.30 (s), | 171.27 (s), |
| 170.74 (s), | 170.19 (s), |
| 168.69 (s), | 157.59 (s), |
| 140.53 (d), | 139.35 (s), |
| 139.18 (s), | 135.76 (d), |
| 134.17 (s), | 131.15 (d) x 2, |
| 130.93 (d), | 130.35 (d), |
| 129.88 (d) x 2, | 129.39 (d) x 2, |
| 128.70 (d), | 128.58 (s), |
| 128.13 (d), | 127.64 (d), |
| 127.53 (d), | 121.99 (d), |
| 116.45 (d) x 2, | 72.76 (d), |
| 70.82 (d), | 62.73 (t), |
| 62.67 (d), | 59.35 (d), |
| 56.33 (d) x 2, | 56.19 (d), |
| 53.36 (d), | 52.24 (d), |
| 40.24 (t), | 37.55 (t), |
| 37.08 (t), | 33.69 (t), |
| 31.57 (t), | 29.93 (q), |
| 24.61 (d), | 23.70 (q), |
| 23.59 (t), | 22.16 (q), |
| 21.36 (q), | 17.12 (q), |
| 14.23 (q) | |

(13) $^1H$ Magnetisches Kernresonanzspektrum: (400 MHz, $CD_3OD$) $\delta$

EP 0 327 009 B1

| 7.86 | (1H, d, J = 16Hz), |
|---|---|
| 7.80 | (1H, br d, J = 8Hz), |
| 7.12-7.42 | (11H, m), |
| 6.77 | (2H, d, J = 8.5Hz), |
| 6.61 | (1H, d, J = 11.5Hz), |
| 5.88 | (1H, dt, J = 7.5 und 11.5Hz), |
| 5.08 | (1H, dd, J = 3.5 und 10Hz), |
| 5.04 | (1H, q, J = 6.5Hz), |
| 4.66 | (1H, dd, J = 3.5 und 13Hz), |
| 4.65 | (1H, d, J = 11.5Hz), |
| 4.56 | (1H, dd, J = 2.5 und 7Hz), |
| 4.48 | (1H, dd, J = 4.5 und 11Hz), |
| 4.46 | (1H, s), |
| 3.88 | (2H, m), |
| 3.64 | (2H, m), |
| 3.51 | (1H, dd, J = 3.5 und 14Hz), |
| 3.17 | (1H, dd, J = 4.5 und 14Hz), |
| 3.01 | (1H, dd, J = 11 und 14Hz), |
| 2.94 | (1H, dd, J = 3.5 und 16Hz), |
| 2.71 | (3H, s), |
| 2.71 | (1H, dd, J = 10 und 16Hz), |
| 2.64 | (1H, dd, J = 13 und 14Hz). |
| 2.04 | (2H, m), |
| 1.43 | (2H, m), |
| 1.28 | (2H, m), |
| 1.20 | (3H, d, J = 6Hz), |
| 0.95 | (3H, d, J = 6.5Hz), |
| 0.87 | (3H, t, J = 7.5Hz), |
| 0.53 | (1H, m), |
| 0.52 | (6H, d, J = 10.5Hz) |

welches das Kultivieren eines WS-9326A- und/oder WS-9326B-produzierenden Stammes, der zur Gattung Streptomyces gehört, in einem Nährstoffmedium und das Gewinnen einer Verbindung, ausgewählt aus WS-9326A und WS-9326B, oder einem Salz davon, aus der resultierenden Nährlösung umfaßt.

2. Verfahren nach Anspruch 1 zur Herstellung einer Verbindung, ausgewählt aus: WS-9326A substituiert mit 1 bis 3 Acylgruppe(n), WS-9326B substituiert mit 1 bis 3 Acylgruppe(n), oder eines Salzes davon, welches das Reagieren einer Verbindung, ausgewählt aus WS-9326A und WS-9326B, oder einem Salz davon mit acylierendem(n) Mittel(n) umfaßt.

3. Verfahren nach Anspruch 1 zur Herstellung von WS-9326A oder eines Salzes davon.

4. Verfahren nach Anspruch 1 zur Herstellung von WS-9326B oder eines Salzes davon.

5. Verfahren nach Anspruch 1 zur Herstellung von WS-9326A substituiert mit 1 bis 3 Acylgruppe(n) oder eines Salzes davon.

6. Verfahren nach Anspruch 5, worin die Acylgruppe nieder Alkanoyl ist.

7. Verfahren nach Anspruch 6 zur Herstellung von Triacetyl-WS-9326A mit folgenden physikalischen und chemischen Eigenschaften:
(1) Form und Farbe: Farbloses Pulver
(2) Farbreaktion:
Positiv: Cersulfat-Reaktion, Schwefelsäure-Reaktion, Joddampf-Reaktion
Negativ: Ninhydrin-Reaktion

67

(3) Löslichkeit:

Löslich:         Methanol, Dimethylsulfoxid
Mäßig löslich:    Chloroform, Diethylether
Unlöslich:      n-Hexan

(4) Schmelzpunkt: 141-143 °C

(5) Spezifische Drehung: $[\alpha]^{23}_D$ : -122 ° (C = 1,0; MeOH)

(6) Ultraviolettes Absorptionsspektrum:

$$\lambda^{MeOH}_{max} = 283 \ nm \ (\mathcal{E} = 32000)$$

(7) Molekularformel: $C_{60}H_{74}N_8O_{16}$

(8)

| Elementaranalyse: | | | |
|---|---|---|---|
| Gef.: | C 60,19; | H 6,42; | N 9,27 |
| Ber. für $C_{60}H_{74}N_8O_{16} \cdot 2H_2O$: | C 60,09; | H 6,56; | N 9,34 |

(9) Molekulargewicht: FAB-MS: m/z 1163,6 $(M + H)^+$

(10) Dünnschichtchromatographie:

| Stationäre Phase | Entwicklungslösungsmittel | Rf Wert |
|---|---|---|
| Silicagel-Platte (Merck Art. 5715) | Chloroform-Methanol (10:1, V/V) | 0,50 |
| | Ethylacetat | 0,12 |

(11) Infrarotes Absorptionsspektrum:

$$\gamma^{KBr}_{max} = 3350, \ 3020, \ 2950, \ 2920, \ 2850, \ 1730,$$
$$1650, \ 1520, \ 1440, \ 1360, \ 1230, \ 1200,$$
$$1160, \ 1100, \ 1060, \ 1040, \ 910 \ cm^{-1}$$

(12) Eigenschaft der Substanz: Neutrale Substanz

(13) $^{13}C$ Magnetisches Kernresonanzspektrum: (100 MHz, $CDCl_3$-$CD_3OH$ (10:1) ) $\delta$

| | |
|---|---|
| 174.20 (s), | 173.23 (s), |
| 173.06 (s), | 171.32 (s), |
| 171.02 (s), | 170.84 (s), |
| 169.79 (s), | 169.59 (s), |
| 169.55 (s), | 168.52 (s), |
| 167.03 (s), | 166.36 (s), |
| 151.02 (s), | 140.74 (d), |
| 138.82 (s), | 138.74 (s), |
| 137.12 (s), | 135.23 (d), |
| 133.75 (s), | 131.31 (s), |
| 130.20 (d), | 129.96 (d) x 2, |
| 129.34 (d), | 129.21 (d) x 2, |
| 128.56 (d) x 2, | 127.24 (d), |
| 126.95 (d), | 126.74 (d), |
| 126.63 (d), | 126.50 (d), |
| 122.10 (d) x 2, | 121.29 (d), |
| 70.99 (d), | 69.22 (d), |
| 63.73 (t), | 58.13 (d), |
| 56.10 (d), | 53.22 (d), |
| 52.66 (d), | 52.18 (d), |
| 49.93 (d), | 39.75 (t), |
| 39.39 (q), | 39.06 (t), |
| 35.57 (t), | 30.65 (t), |
| 24.26 (d), | 23.15 (q), |
| 22.79 (t), | 21.42 (q), |
| 21.21 (q), | 20.99 (q), |
| 20.83 (q), | 17.05 (q), |
| 16.18 (q), | 13.82 (q) |

(14) $^1$H Magnetisches Kernresonanzspektrum: (400 MHz, $CDCl_3$-$CD_3OH$ (10:1)) $\delta$

| | |
|---|---|
| 8.25 | (1H, d, J=8Hz), |
| 8.02 | (1H, d, J=8Hz), |
| 7.88 | (1H, d, J=16Hz), |
| 7.86 | (1H, d, J=8Hz), |
| 7.70 | (1H, d, J=6Hz), |
| 7.61 | (1H, d, J=8Hz), |
| 7.45 | (1H, d, J=7Hz), |
| 7.32-7.15 | (6H, m), |
| 7.03 | (2H, d, J=8Hz), |
| 7.00-6.94 | (3H, m), |
| 6.88-6.79 | (4H, m), |
| 6.70 | (1H, s), |
| 6.49 | (1H, d, J=12Hz), |
| 5.76 | (1H, dt, J=12 und 7.5Hz), |
| 5.54 | (1H, breit s), |
| 5.50-5.45 | (2H, m), |
| 4.93 | (1H, m), |
| 4.75 | (1H, m) |
| 4.65-4.56 | (2H, m), |
| 4.46 | (1H, dd, J=6 und 11Hz), |
| 4.31 | (1H, t, J=6Hz), |
| 4.22 | (1H, m), |
| 4.18 | (1H, dd, J=8 und 11Hz), |
| 3.56 | (3H, s), |
| 2.90 | (1H, dd, J=6 und 16Hz), |
| 2.85-2.80 | (2H, m), |
| 2.56 | (1H, dd, J=4 und 16Hz), |
| 2.26 | (3H, s), |
| 2.00 | (3H, s), |
| 1.96-1.89 | (2H, m), |
| 1.85 | (3H, s), |
| 1.58 | (1H, m), |
| 1.35 | (3H, d, J=6Hz), |
| 1.32-1.20 | (3H, m), |
| 1.07 | (3H, d, J=6Hz), |
| 0.84 | (1H, m), |
| 0.72 | (3H, d, J=6Hz), |
| 0.71 | (3H, t, J=7.5Hz), |
| 0.65 | (3H, d, J=6Hz) |

8. Modifizierung des Verfahrens nach jedem der Ansprüche 1 bis 7, die dadurch charakterisiert ist, daß eine Verbindung, hergestellt nach dem Verfahren nach jedem der Ansprüche 1 bis 7, oder ein pharmazeutisch verträgliches Salz davon durch Zusammenmischen oder Präsentation der genannten Verbindung mit einem pharmazeutisch verträglichen Verdünnungsmittel oder Träger in eine pharmazeutisch verträgliche Form gebracht wird.

9. Biologisch reine Kultur des Mikroorganismus Streptomyces violaceoniger Nr. 9326 (FERM BP-1667).

**Revendications**
**Revendications pour les Etats contractants suivants : AT, BE, CH, DE, FR, GB, IT, LI, LU, NL, SE**

1. Composé choisi parmi les WS-9326A, WS-9326B, WS-9326A substitué avec 1 à 3 groupes acyle et WS-9326B substitué avec 1 à 3 groupes acyle, et un de leurs sels pharmaceutiquement acceptable, dans lequel
   (i) WS-9326A a les propriétés physiques et chimiques suivantes :
      (1) forme et couleur :
         poudre incolore
      2) réaction aux couleurs :
         Positive :    réaction au sulfate de cérium, réaction à la vapeur d'iode réaction au chlorure ferriqueferrocyanure de potassium
         Négative :    réaction à la ninhydrine, réaction de Molish, réaction au chlorure ferrique, réaction de Ehrlich, réaction de Pauli.
      (3) Solubilité :
         Soluble :                  méthanole, éthanol
         Modérément soluble :       acétone, acétate d'éthyle
         Insoluble :                eau, chloroforme
      (4) Point de fusion : 187-190 °C
      (5) Rotation spécifique :
         $[\alpha]_D^{23}$ : -84 ° (C = 1,0; MeOH)
      (6) Spectre d'absorption de l'ultraviolet :

$$\lambda \, ^{\text{MeOH}}_{\text{Max}} = 280 \text{ nm } (\varepsilon=34.700)$$

      (7) Spectre d'absorption de l'infrarouge :

$$\upsilon \, ^{\text{KBr}}_{\text{Max}} = 3300,3050, \ 2950, \ 2920, \ 2860,1730,$$
$$1650, \ 1610, \ 1560, \ 1540, \ 1530, \ 1510,$$
$$1440, \ 1380, \ 1340, \ 1280, \ 1240, \ 1170,$$
$$1110, \ 1080, \ 1060, \ 1040, \ 970, \ 920,$$
$$880, \ 860, \ 830 \text{ cm}^{-1}$$

      (8)

| Analyse élémentaire : | | | |
|---|---|---|---|
| Trouvé : | C 60,18, | H 6,61, | N 10,32 |
| Calc. pour $C_{54}H_{68}N_8O_{13}.2H_2O$ : | C 60,43, | H 6,76, | N 10,44 |

      (9) Chromatographie sur couche mince :

71

| Phase stationnaire | Solvant de développement | Valeur Rf |
|---|---|---|
| Plaque de gel de silice (Merck Art 5715 Plaque RP-18 | chloroforme-méthanol méthanol-eau (5/1, V/V) (8:2, V/V) | 0,38 0,46 |

(10) Formule moléculaire : $C_{54}H_{68}N_8O_{13}$

(11) Masse moléculaire :

FAB-MS : m/z 1037 $(M+H)^+$

(12) Propriété de la substance :

substance acide

(13) Spectre de résonance magnétique nucléaire $^{13}C$ (100 MHz, $CD_3OD$) $\delta$

| | |
|---|---|
| 175,69 (s), | 174,70 (s), |
| 173,73 (s), | 173,38 (s), |
| 172,89 (s), | 171,04 (s), |
| 170,45 (s), | 167,79 (s), |
| 167,15 (s), | 159,20 (s), |
| 140,05 (d), | 139,12 (s), |
| 138,71 (s), | 135,27 (d), |
| 134,85 (s), | 132,11 (d), |
| 132,03 (s), | 131,69 (d) x 2, |
| 130,70 (d), | 129,90 (d), |
| 129,61 (d) x 2, | 129,22 (d) x 2, |
| 128,55 (d), | 128,04 (d), |
| 127,99 (d), | 127,38 (d), |
| 126,09 (s), | 123,70 (d), |
| 115,63 (d) x 2, | 73,46 (d), |
| 71,34 (d), | 62,80 (t), |
| 59,53 (d), | 56,91 (d), |
| 56,76 (d), | 55,55 (d), |
| 53,64 (d), | 52,10 (d), |
| 39,85 (t), | 37,18 (t), |
| 37,09 (t), | 34,58 (q), |
| 31,37 (t), | 24,56 (d), |
| 23,63 (t), | 22,71 (q), |
| 22,52 (q), | 21,17 (q), |
| 17,19 (q), | 14,13 (q) |

(14) Spectre de résonance magnétique nucléaire $^1H$ (400 MHz, $CD_3OD$) $\delta$

| 7,80 | (1H, d, J = 8Hz), |
|---|---|
| 7,67 | (1H, d, J = 16Hz), |
| 7,45-7.14 | (9H, m), |
| 7,06 | (2H, d, J = 8Hz), |
| 6,83 | (1H, s), |
| 6,65 | (2H, d, J = 8Hz), |
| 6,59 | (1H, d, J = 12Hz), |
| 5,88 | (1H, dt, J = 12 et 7Hz), |
| 5,55 | (1H, m), |
| 5,35 | (1H, signal large), |
| 5,10 | (1H, dd, J = 3 et 9.5Hz), |
| 4,68 | (1H, d, J = 10Hz), |
| 4,55 | (1H, t, J = 6Hz), |
| 4,48 | (1H, dd, J = 3 et 12Hz), |
| 3,92 | (2H, d, J = 6Hz), |
| 3,70 | (1H, t, J = 7.5Hz), |
| 3,62 | (1H, m), |
| 3,46 | (1H, dd, J = 3 et 14Hz), |
| 2,94 | (1H, dd, J = 3 et 16Hz), |
| 2,89 | (3H, s), |
| 2,74 | (1H, dd, J = 9.5 et 16Hz), |
| 2,69 | (1H, dd, J = 12 et 14Hz), |
| 2,14 | (2H, m), |
| 1,5-1,4 | (2H, m), |
| 1,20 | (3H, d, J = 6Hz) |
| 1,08 | (3H, d, J = 6Hz), |
| 1,0-0,8 | (2H, m), |
| 0,91 | (3H, t, J = 7Hz), |
| 0,6 | (1H, m) |
| 0,53 | (3H, d, J = 6Hz), |
| 0,51 | (3H, d, J = 6Hz) |

(ii) Le WS-9362B a les propriétés physiques et chimiques suivantes :

(1) Forme et couleur : poudre amorphe incolore

(2) réaction aux couleurs :

Positive : réaction au sulfate de cérium, réaction à la vapeur d'iode

Négative : réaction à la ninhydrine

(3) Solubilité :

Soluble : méthanole

Modérément soluble : éthanol

Insoluble : eau, acétone, acétate d'éthyle, chloroforme

(4) Point de fusion : 165-170 °C (déc.)

(5) Rotation spécifique : $[\alpha]_D^{23}$ : -64 ° (C = 1,0; MeOH)

(6) Spectre d'absorption de l'ultraviolet :

$$\lambda_{max}^{MeOH} = 283 \text{ nm } (\varepsilon = 27.000)$$

(7) Formule moléculaire : $C_{54}H_{70}N_8O_{13}$

(8)

| Analyse élémentaire : | | | |
|---|---|---|---|
| Trouvé : | C 59,97, | H 6,87, | N 10,29 |
| Calc. pour : $C_{54}H_{70}N_8O_{13} \cdot 2H_2O$ : | C 60,32, | H 6,94, | N 10,42 |

(9) Masse moléculaire :

FAB-MS :     m/z 1061,6 $(M + Na)^+$

(10) Chromatographie sur couche mince :

| Phase stationnaire | Solvant de développement | Valeur Rf |
|---|---|---|
| Plaque de gel de Silice (Merck Art 5715) | Chloroformeméthanole (5:1, V/V) | 0,38 |
| Plaque RP-18 | méthanole-eau (8:2, V/V) | 0,25 |

(11) Spectre d'absorption de l'infrarouge :

$$\nu_{max}^{KBr} = 3300, 3050, 2950, 1735, 1660, 1530,$$
$$1510, 1450, 1400, 1380, 1340, 1260,$$
$$1220, 1080, 980, 920\ cm^{-1}$$

(12) Spectre de résonance magnétique nucléaire $13_C$ (100 MHz, $CD_3OD$) $\delta$

| | |
|---|---|
| 174,99 (s), | 174,54 (s), |
| 173,60 (s), | 173,41 (s), |
| 173,30 (s), | 171,27 (s), |
| 170,74 (s), | 170,19 (s), |
| 168,69 (s), | 157,59 (s), |
| 140,53 (d), | 139,35 (s), |
| 139,18 (s), | 135,76 (d), |
| 134,17 (s), | 131,15 (d) x 2, |
| 130,93 (d), | 130,35 (d), |
| 129,88 (d) x 2, | 129,39 (d) x 2, |
| 128,70 (d), | 128,58 (s), |
| 128,13 (d), | 127,64 (d), |
| 127,53 (d), | 121,99 (d), |
| 116,45 (d) x 2, | 72,76 (d), |
| 70,82 (d), | 62,73 (t), |
| 62,67 (d), | 59,35 (d), |
| 56,33 (d) x 2, | 56,19 (d), |
| 53,36 (d), | 52,24 (d), |
| 40,24 (t), | 37,55 (t), |
| 37,08 (t), | 33,69 (t), |
| 31,57 (t), | 29,93 (q), |
| 24,61 (d), | 23,70 (q), |
| 23,59 (t), | 22,16 (q), |
| 21,36 (q), | 17,12 (q), |
| 14,23 (q) | |

(13) Spectre de résonance magnétique nucléaire $1_H$ : (400 MHz, $CD_3OD$) $\delta$

| | |
|---|---|
| 7,86 | (1H, d, J = 16Hz), |
| 7,80 | (1H, br d, J = 8Hz), |
| 7,12-7.42 | (11H, m), |
| 6,77 | (2H, d, J = 8.5Hz), |
| 6,61 | (1H, d, J = 11.5Hz), |
| 5,88 | (1H, dt, J = 7.5 et 11.5Hz), |
| 5,08 | (1H, dd, J = 3.5 et 10Hz), |
| 5,04 | (1H, q, J = 6.5Hz), |
| 4,66 | (1H, dd, J = 3.5 et 13Hz), |
| 4,65 | (1H, d, J = 11.5Hz), |
| 4,56 | (1H, dd, J = 2.5 et 7Hz), |
| 4,48 | (1H, dd, J = 4.5 et 11Hz), |
| 4,46 | (1H, s), |
| 3,88 | (2H, m), |
| 3,64 | (2H, m), |
| 3,51 | (1H, dd, J = 3.5 et 14Hz), |
| 3,17 | (1H, dd, J = 4.5 et 14Hz), |
| 3,01 | (1H, dd, J = 11 et 14Hz), |
| 2,94 | (1H, dd, J = 3.5 et 16Hz), |
| 2,71 | (3H, s), |
| 2,71 | (1H, dd, J = 10 et 16Hz), |
| 2,64 | (1H, dd, J = 13 et 14Hz), |
| 2,04 | (2H, m), |
| 1,43 | (2H, m), |
| 1,28 | (2H, m), |
| 1,20 | (3H, d, J = 6Hz), |
| 0,95 | (3H, d, J = 6.5Hz), |
| 0,87 | (3H, t, J = 7.5Hz), |
| 0,53 | (1H, m), |
| 0,52 | (6H, d, J = 10.5Hz) |

**2.** Composé selon la revendication 1, dans lequel le groupe acyle est un groupe alkanoyl inférieur.

**3.** Composé selon la revendication 2, qui est le triacéthyl-WS-9326A ayant les propriétés physiques et chimiques suivantes :

(1) Forme et couleur : poudre incolore

(2) Réaction aux couleurs :

Positive : réaction au sulfate de cérium, réaction à l'acide sulfurique, réaction à la vapeur d'iode

Négative : réaction à la ninhydrine

(3) Solubilité :

Soluble : méthanole, sulfoxyde de diméthyle

Modérément soluble : chloroforme, éther diéthylique

Insoluble : n-héxane

(4) Point de fusion : 141-143°C

(5) Rotation spécifique : $[\alpha]_D^{23}$ : -122° (C = 1,0; MeOH)

(6) Spectre d'absorption de l'ultraviolet :

$$\lambda \, \frac{MeOH}{max} = 283 \ nm \ (\varepsilon = 32.000)$$

(7) Formule moléculaire : $C_{60}H_{74}N_8O_{16}$

(8)

| Analyse élémentaire : | | | |
|---|---|---|---|
| Trouvé : | C 60,19, | H 6,42, | N 9,27 |
| Calc. pour $C_{60}H_{74}N_8O_{16}.2H_2O$ : | C 60,09, | H 6,56, | N 9,34 |

(9) Masse moléculaire :

FAB-MS : m/z 1163,6 $(M+H)^+$

(10) Chromatographie sur couche mince :

| Phase stationnaire | Solvant de développement | Valeur Rf |
|---|---|---|
| Plaque de gel de Silice (Merck Art 5715) | Chloroformeméthanole (10:1, V/V) acétate d'éthyle | 0,50 0,12 |

(11) Spectre d'absorption de l'infrarouge :

$$\nu_{max}^{KBr} = 3350, 3020, 2950, 2920, 2850, 1730,$$
$$1650, 1520, 1440, 1360, 1230, 1200,$$
$$1160, 1100, 1060, 1040, 910 \; cm^{-1}$$

(12) Propriété de la substance :

substance neutre

(13) Spectre de résonance magnétique nucléaire $13_C$ (100 MHz, $CDC\ell_3$-$CD_3OH$ (10:1)) $\delta$

| | |
|---|---|
| 174,20 (s), | 173,23 (s), |
| 173,06 (s), | 171,32 (s), |
| 171,02 (s), | 170,84 (s), |
| 169,79 (s), | 169,59 (s), |
| 169,55 (s), | 168,52 (s), |
| 167,03 (s), | 166,36 (s), |
| 151,02 (s), | 140,74 (d), |
| 138,82 (s), | 138,74 (s), |
| 137,12 (s), | 135,23 (d), |
| 133,75 (s), | 131,31 (s), |
| 130,20 (d), | 129,96 (d) x 2, |
| 129,34 (d), | 129,21 (d) x 2, |
| 128,56 (d) x 2, | 127,24 (d), |
| 126,95 (d), | 126,74 (d), |
| 126,63 (d), | 126,50 (d), |
| 122,10 (d) x 2, | 121,29 (d), |
| 70,99 (d), | 69,22 (d), |
| 63,73 (t), | 58,13 (d), |
| 56,10 (d), | 53,22 (d), |
| 52,66 (d), | 52,18 (d), |
| 49,93 (d), | 39,75 (t), |
| 39,39 (q), | 39,06 (t), |
| 35,57 (t), | 30,65 (t), |
| 24,26 (d), | 23,15 (q), |
| 22,79 (t), | 21,42 (q), |
| 21,21 (q), | 20,99 (q), |
| 20,83 (q), | 17,05 (q), |
| 16,18 (q), | 13,82 (q) |

(14) Spectre de résonance magnétique nucléaire $^1$H (400 MHz, CDC$\ell_3$-CD$_3$OH (10:1)) $\delta$

```
8,25        (1H, d, J=8Hz),
8,02        (1H, d, J=8Hz),
7,88        (1H, d, J=16Hz),
7,86        (1H, d, J=8Hz),
7,70        (1H, d, J=6Hz),
7,61        (1H, d, J=8Hz),
7,45        (1H, d, J=7Hz),
7,32-7,15 (6H, m),
7,03        (2H, d, J=8Hz),
7,00-6,94 (3H, m),
6,88-6,79 (4H, m),
6,70        (1H, s),
6,49        (1H, d, J=12Hz),
5,76        (1H, dt, J=12  et 7.5Hz),
5,54        (1H, broad s),
5,50-5,45 (2H, m),
4,93        (1H, m),
4,75        (1H, m)
4,65-4,56 (2H, m),
4,46        (1H, dd, J=6  et 11Hz),
4,31        (1H, t, J=6Hz),
4,22        (1H, m),
4,18        (1H, dd, J=8  et 11Hz),
3,56        (3H, s),
2,90        (1H, dd, J=6  et 16Hz),
2,85-2,80 (2H, m),
2,56        (1H, dd, J=4  et 16Hz),
2,26        (3H, s),
2,00        (3H, s),
1,96-1,89 (2H, m),
1,85        (3H, s),
1,58        (1H, m),
1,35        (3H, d, J=6Hz),
1,32-1,20 (3H, m),
1,07        (3H, d, J=6Hz),
0,84        (1H, m),
0,72        (3H, d, J=6Hz),
0,71        (3H, t, J=7.5Hz),
0,65        (3H, d, J=6Hz)
```

4. Procédé pour la préparation d'un composé choisi parmi WS-9326A et 9326B selon la revendication 1, où un de leurs sels, qui comprend la mise en culture d'une souche de production de WS-9326A et/ou WS-9326B appartenant au genre <u>Streptomyces</u> dans un milieu nutritif and la récupération d'un composé choisi Parmi WS-9326A et WS-9326B, ou un de leurs sels à partir du bouillon de culture obtenu.

**5.** Procédé pour la production d'un composé choisi parmi WS-9326A substitué avec 1 à 3 groupes acyles et WS-9326B substitué avec 1 à 3 groupes à cyle selon la revendications 1, où un de leurs sels, qui comprend le fait de faire réagir un composé choisi parmi WS-9326A et WS-9326B, où un de leurs sels avec un ou plusieurs agents d'acylation.

**6.** Composition pharmaceutique comportant un composé choisi parmi WS-9326A, WS-9326B, WS-9326A susbtitué avec 1 à 3 groupes acyle et WS-9326B susbtitué avec 1 à 3 groupes acyle selon la revendication 1, ou un sel pharmaceutiquement acceptables, comme ingrédient actif, en association avec un support ou excipient pharmaceutiquement acceptable, sensiblement non toxique.

**7.** Utilisation d'un composé choisi parmi WS-9326A, WS-9326B, WS-9326A substitué avec 1 à 3 groupes acyle et WS-9326B susbstitué avec 1 à 3 groupes acyle selon la revendication 1, ou un de leurs sels pharmaceutiquement acceptables pour le traitement et la prévention de l'asthme.

**8.** Culture biologiquement pure du microorganisme <u>Streptomyces</u> <u>violaceoniger</u> No. 9326 (FERM BP-1667)

**Revendications pour l'Etat contractant suivant : ES**

**1.** Procédé pour la production d'un composé choisi parmi les WS-9326A, WS-9326B, WS-9326A substitué avec 1 à 3 groupes acyle et WS-9326B substitué avec 1 à 3 groupes acyle, et un de leurs sels, dans lequel

(i) WS-9326A a les propriétés physiques et chimiques suivantes :

(1) forme et couleur :

poudre incolore

2) réaction aux couleurs :

Positive : réaction au sulfate de cérium, réaction à la vapeur d'iode réaction au chlorure ferrique-ferrocyanure de potassium

Négative : réaction à la ninhydrine, réaction de Molish, réaction au chlorure ferrique, réaction d'Ehrlich, réaction de Pauli.

(3) Solubilité :

Soluble : méthanol, éthanol

Modérément soluble : acétone, acétate d'éthyle

Insoluble : eau, chloroforme

(4) Point de fusion : 187-190 °C

(5) Rotation spécifique :

$[\alpha]_D^{23}$ : -84 ° (C = 1,0; MeOH)

(6) Spectre d'absorption de l'ultraviolet :

$$\lambda \, {}^{MeOH}_{Max} = 280 \ nm \ (\varepsilon = 34.700)$$

(7) Spectre d'absorption de l'infrarouge :

$$\upsilon \begin{array}{c} KBr \\ Max \end{array} = 3300, 3050, 2950, 2920, 2860, 1730,$$
$$1650, 1610, 1560, 1540, 1530, 1510,$$
$$1440, 1380, 1340, 1280, 1240, 1170,$$
$$1110, 1080, 1060, 1040, 970, 920,$$
$$880, 860, 830 \text{ cm}^{-1}$$

(8)

| Analyse élémentaire : | | | |
|---|---|---|---|
| Trouvé : | C 60,18, | H 6,61, | N 10,32 |
| Calc. pour $C_{54}H_{68}N_8O_{13} \cdot 2H_2O$ : | C 60,43, | H 6,76, | N 10,44 |

(9) Chromatographie sur couche mince :

| Phase stationnaire | Solvant de développement | Valeur Rf |
|---|---|---|
| Plaque de gel de silice (Merck Art 5715 plaque RP-18 | chloroforme-méthanol (5/1, V/V) méthanol-eau (8:2, V/V) | 0,38 0,46 |

(10) Formule moléculaire : $C_{54}H_{68}N_8O_{13}$
(11) Masse moléculaire :
     FAB-MS :      m/z 1037 $(M+H)^+$
(12) Propriété de la substance :
       substance acide
(13) Spectre de résonance magnétique nucléaire $^{13}C$ (100 MHz, $CD_3OD$) δ

| | |
|---|---|
| 175,69 (s), | 174,70 (s), |
| 173,73 (s), | 173,38 (s), |
| 172,89 (s), | 171,04 (s), |
| 170,45 (s), | 167,79 (s), |
| 167,15 (s), | 159,20 (s), |
| 140,05 (d), | 139,12 (s), |
| 138,71 (s), | 135,27 (d), |
| 134,85 (s), | 132,11 (d), |
| 132,03 (s), | 131,69 (d) x 2, |
| 130,70 (d), | 129,90 (d), |
| 129,61 (d) x 2, | 129,22 (d) x 2, |
| 128,55 (d), | 128,04 (d), |
| 127,99 (d), | 127,38 (d), |
| 126,09 (s), | 123,70 (d), |
| 115,63 (d) x 2, | 73,46 (d), |
| 71,34 (d), | 62,80 (t), |
| 59,53 (d), | 56,91 (d), |
| 56,76 (d), | 55,55 (d), |
| 53,64 (d), | 52,10 (d), |
| 39,85 (t), | 37,18 (t), |
| 37,09 (t), | 34,58 (q), |
| 31,37 (t), | 24,56 (d), |
| 23,63 (t), | 22,71 (q), |
| 22,52 (q), | 21,17 (q), |
| 17,19 (q), | 14,13 (q) |

(14) Spectre de résonance magnétique nucléaire $^1$H (400 MHz, $CD_3OD$) $\delta$

| | |
|---|---|
| 7,80 | (1H, d, J = 8Hz), |
| 7,67 | (1H, d, J = 16Hz), |
| 7,45-7,14 | (9H, m), |
| 7,06 | (2H, d, J = 8Hz), |
| 6,83 | (1H, s), |
| 6,65 | (2H, d, J = 8Hz), |
| 6,59 | (1H, d, J = 12Hz), |
| 5,88 | (1H, dt, J = 12 et 7Hz), |
| 5,55 | (1H, m), |
| 5,35 | (1H, signal large ), |
| 5,10 | (1H, dd, J = 3 et 9.5Hz), |
| 4,68 | (1H, d, J = 10Hz), |
| 4,55 | (1H, t, J = 6Hz), |
| 4,48 | (1H, dd, J = 3 et 12Hz), |
| 3,92 | (2H, d, J = 6Hz), |
| 3,70 | (1H, t, J = 7.5Hz), |
| 3,62 | (1H,m), |
| 3,46 | (1H, dd, J = 3 et 14Hz), |
| 2,94 | (1H, dd, J = 3 et 16Hz), |
| 2,89 | (3H, s), |
| 2,74 | (1H, dd, J = 9.5 et 16Hz), |
| 2,69 | (1H, dd, J = 12 et 14Hz), |
| 2,14 | (2H, m), |
| 1,5-1,4 | (2H, m), |
| 1,20 | (3H, d, J = 6Hz) |
| 1,08 | (3H, d, J = 6Hz), |
| 1,0-0,8 | (2H, m), |
| 0,91 | (3H, t, J = 7Hz), |
| 0,6 | (1H, m) |
| 0,53 | (3H, d, J = 6Hz), |
| 0,51 | (3H, d, J = 6Hz) |

(ii) Le WS-9326B a les propriétés physiques et chimiques suivantes :

(1) Forme et couleur : poudre amorphe incolore

(2) réaction aux couleurs :

Positive :  réaction au sulfate de cérium, réaction à la vapeur diode

Négative :  réaction à la ninhydrine

(3) Solubilité :

Soluble :  méthanol

Modérément soluble :  éthanol

Insoluble :  eau, acétone, acétate d'éthyle, chloroforme

(4) Point de fusion : 165-170 °C (déc.)

(5) Rotation spécifique : $[\alpha]_D^{23}$ : -64° (C = 1,0; MeOH)

(6) Spectre d'absorption de l'ultraviolet :

$$\lambda \begin{smallmatrix} MeOH \\ Max \end{smallmatrix} = 283 \text{ nm } (\varepsilon = 27.000)$$

(7) Formule moléculaire : $C_{54}H_{70}N_8O_{13}$

(8)

| Analyse élémentaire : | | | |
|---|---|---|---|
| Trouvé : | C 59,97, | H 6,87, | N 10,29 |
| Calc. pour : $C_{54}H_{70}N_8O_{13}.2H_2O$ : | C 60,32, | H 6,94, | N 10,42 |

(9) Masse moléculaire :

FAB-MS : m/z 1061,6 $(M + Na)^+$

(10) Chromatographie sur couche mince :

| Phase stationnaire | Solvant de développement | Valeur Rf |
|---|---|---|
| Plaque de gel de Silice (Merck Art 5715) | Chloroformeméthanol (5:1, V/V) | 0,38 |
| Plaque RP-18 | méthanol-eau (8:2, V/V) | 0,25 |

(11) Spectre d'absorption de l'infrarouge :

$$\nu_{max}^{KBr} = 3300, 3050, 2950, 1735, 1660, 1530,$$
$$1510, 1450, 1400, 1380, 1340, 1260,$$
$$1220, 1080, 980, 920 \ cm^{-1}$$

(12) Spectre de résonance magnétique nucléaire $13_C$ (100 MHz, $CD_3OD$) $\delta$

| | |
|---|---|
| 174,99 (s), | 174, 54 (s), |
| 173,60 (s), | 173,41 (s), |
| 173,30 (s), | 171,27 (s), |
| 170,74 (s), | 170,19 (s), |
| 168,69 (s), | 157,59 (s), |
| 140,53 (d), | 139,35 (s), |
| 139,18 (s), | 135,76 (d), |
| 134,17 (s), | 131,15 (d) x 2, |
| 130,93 (d), | 130,35 (d), |
| 129,88 (d) x 2, | 129,39 (d) x 2, |
| 128,70 (d), | 128,58 (s), |
| 128,13 (d), | 127,64 (d), |
| 127,53 (d), | 121,99 (d), |
| 116,45 (d) x 2, | 72,76 (d), |
| 70,82 (d), | 62,73 (t), |
| 62,67 (d), | 59,35 (d), |
| 56,33 (d) x 2, | 56,19 (d), |
| 53,36 (d), | 52,24 (d), |
| 40,24 (t), | 37,55 (t), |
| 37,08 (t), | 33,69 (t), |
| 31,57 (t), | 29,93 (q), |
| 24,61 (d), | 23,70 (q), |
| 23,59 (t), | 22,16 (q), |
| 21,36 (q), | 17,12 (q), |
| 14,23 (q) | |

(13) Spectre de résonance magnétique nucléaire $1_H$ : (400 MHz, $CD_3OD$) $\delta$

| | |
|---|---|
| 7,86 | (1H, d, J = 16Hz), |
| 7,80 | (1H, br d, J = 8Hz), |
| 7,12-7.42 | (11H, m), |
| 6,77 | (2H, d, J = 8.5Hz), |
| 6,61 | (1H, d, J = 11.5Hz), |
| 5,88 | (1H, dt, J = 7.5 et 11.5Hz), |
| 5,08 | (1H, dd, J = 3.5 et 10Hz), |
| 5,04 | (1H, q, J = 6.5Hz), |
| 4,66 | (1H, dd, J = 3.5 et 13Hz), |
| 4,65 | (1H, d, J = 11.5Hz), |
| 4,56 | (1H, dd, J = 2.5 et 7Hz), |
| 4,48 | (1H, dd, J = 4.5 et 11Hz), |
| 4,46 | (1H, s), |
| 3,88 | (2H, m), |
| 3,64 | (2H, m), |
| 3,51 | (1H, dd, J = 3.5 et 14Hz), |
| 3,17 | (1H, dd, J = 4.5 et 14Hz), |
| 3,01 | (1H, dd, J = 11 et 14Hz), |
| 2,94 | (1H, dd, J = 3.5 et 16Hz), |
| 2,71 | (3H, s), |
| 2,71 | (1H, dd, J = 10 et 16Hz), |
| 2,64 | (1H, dd, J = 13 et 14Hz), |
| 2,04 | (2H, m), |
| 1,43 | (2H, m), |
| 1,28 | (2H, m), |
| 1,20 | (3H, d, J = 6Hz), |
| 0,95 | (3H, d, J = 6.5Hz), |
| 0,87 | (3H, t, J = 7.5Hz), |
| 0,53 | (1H, m), |
| 0,52 | (6H, d, J = 10.5Hz) |

qui comprend la mise en culture d'une souche de production de WS-9326A et/ou WS-9326B appartenant au genre Streptomyces dans un milieu nutritif et la récupération d'un composé choisi parmi WS-9326A et WS-9326B, ou un de leurs sels, à partir du bouillon de culture obtenu.

2. Procédé selon la revendication 1, pour la production d'un composé choisi parmi: WS-9326A substitué avec 1 à 3 groupes acyle et WS-9326B susbtitué avec 1 à 3 groupes acyle, ou un de leurs sels, qui comprend la mise en réaction d'un composé choisi parmi WS-9326A et WS-9326B, ou un de leurs sels avec un ou plusieurs agents d'acylation.

3. Procédé selon la revendication 1, pour la production de WS-9326A ou un de ses sels.

4. Procédé selon la revendication 1, pour la production de WS-9326B ou un de ses sels.

5. Procédé selon la revendication 1, pour la production de WS-9326A susbtitué avec 1 à 3 groupes acyle ou un de ses sels.

6. Procédé selon la revendication 5, dans lequel le groupe acyle est un groupe alkanoyl inférieur.

7. Procédé selon la revendication 6, pour la production de triacétyl-WS-9326A ayant les prorpiétés physiques et chimiques suivantes :
   (1) Forme et couleur : poudre incolore
   (2) réaction aux couleurs :
   Positive : réaction au sulfate de cérium, réaction à l'acide sulfurique réaction à la vapeur d'iode
   Négative : réaction à la ninhydrine

84

(3) Solubilité :

    Soluble :                 méthanol, sulfoxyde de diméthyle

    Modérément soluble :     chloroforme, éther diéthylique

    Insoluble :            n-héxane

(4) Point de fusion : 141-143 °C

(5) Rotation spécifique : $[\alpha]_D^{23}$ : -122° (C = 1,0; MeOH)

(6) Spectre d'absorption de l'ultraviolet :

$$\lambda_{max}^{MeOH} = 283 \text{ nm } (\varepsilon=32.000)$$

(7) Formule moléculaire : $C_{60}H_{74}N_8O_{16}$

(8)

| Analyse élémentaire : | | | |
|---|---|---|---|
| Trouvé : | C 60,19, | H 6,42, | N 9,27 |
| Calc. pour : $C_{60}H_{74}N_8O_{16}.2H_2O$ : | C 60,09, | H 6,56, | N 9,34 |

(9) Masse moléculaire :

    FAB-MS :     m/z 1163,6 $(M + H)^+$

(10) Chromatographie sur couche mince :

| Phase stationnaire | Solvant de développement | Valeur Rf |
|---|---|---|
| Plaque de gel de Silice (Merck Art 5715) | Chloroformeméthanol (10:1, V/V) acétate d'éthyle | 0,50 0,12 |

(11) Spectre d'absorption de l'infrarouge :

$$\nu_{max}^{KBr} = 3350, 3020, 2950, 2920, 2850, 1730,$$
$$1650, 1520, 1440, 1360, 1230, 1200,$$
$$1160, 1100, 1060, 1040, 910 \text{ cm}^{-1}$$

(12) Propriété de la substance :

    substance neutre

(13) Spectre de résonnance magnétique nucleaire $13_C$ : (100 MHz, $CDC\ell_3$-$CD_3OH$ (10:1)) $\delta$

| | |
|---|---|
| 174,20 (s), | 173,23 (s), |
| 173,06 (s), | 171,32 (s), |
| 171,02 (s), | 170,84 (s), |
| 169,79 (s), | 169,59 (s), |
| 169,55 (s), | 168,52 (s), |
| 167,03 (s), | 166,36 (s), |
| 151,02 (s), | 140,74 (d), |
| 138,82 (s), | 138,74 (s), |
| 137,12 (s), | 135,23 (d), |
| 133,75 (s), | 131,31 (s), |
| 130,20 (d), | 129,96 (d) x 2, |
| 129,34 (d), | 129,21 (d) x 2, |
| 128,56 (d) x 2, | 127,24 (d), |
| 126,95 (d), | 126,74 (d), |
| 126,63 (d), | 126,50 (d), |
| 122,10 (d) x 2, | 121,29 (d), |
| 70,99 (d), | 69,22 (d), |
| 63,73 (t), | 58,13 (d), |
| 56,10 (d), | 53,22 (d), |
| 52,66 (d), | 52,18 (d), |
| 49,93 (d), | 39,75 (t), |
| 39,39 (q), | 39,06 (t), |
| 35,57 (t), | 30,65 (t), |
| 24,26 (d), | 23,15 (q), |
| 22,79 (t), | 21,42 (q), |
| 21,21 (q), | 20,99 (q), |
| 20,83 (q), | 17,05 (q), |
| 16,18 (q), | 13,82 (q) |

(14) Spectre de résonance magnétique nucléaire $^1$H : (400 MHz, $CDCl_3$-$CD_3OH$ (10:1)) $\delta$

```
8,25        (1H, d, J=8Hz),
8,02        (1H, d, J=8Hz),
7,88        (1H, d, J=16Hz),
7,86        (1H, d, J=8Hz),
7,70        (1H, d, J=6Hz),
7,61        (1H, d, J=8Hz),
7,45        (1H, d, J=7Hz),
7,32-7,15 (6H, m),
7,03        (2H, d, J=8Hz),
7,00-6,94 (3H, m),
6,88-6,79 (4H, m),
6,70        (1H, s),
6,49        (1H, d, J=12Hz),
5,76        (1H, dt, J=12 et 7.5Hz),
5,54        (1H, broad s),
5,50-5.45 (2H, m),
4,93        (1H, m),
4,75        (1H, m)
4,65-4.56 (2H, m),
4,46        (1H, dd, J=6 et  11Hz),
4,31        (1H, t, J=6Hz),
4,22        (1H, m),
4,18        (1H, dd, J=8 et 11Hz),
3,56        (3H, s),
2,90        (1H, dd, J=6 et 16Hz),
2,85-2,80 (2H, m),
2,56        (1H, dd, J=4 et 16Hz),
2,26        (3H, s),
2,00        (3H, s),
1,96-1,89 (2H, m),
1,85        (3H, s),
1,58        (1H, m),
1,35        (3H, d, J=6Hz),
1,32-1,20 (3H, m),
1,07        (3H, d, J=6Hz),
0,84        (1H, m),
0,72        (3H, d, J=6Hz),
0,71        (3H, t, J=7.5Hz),
0,65        (3H, d, J=6Hz)
```

**Revendications pour l'Etat contractant suivant : GR**

1.  Procédé pour la production d'un composé choisi parmi les WS-9326A, WS-9326B, WS-9326A substitué avec 1 à 3 groupes acyle et WS-9326B substitué avec 1 à 3 groupes acyle, ou un de leurs sels,

(i) WS-9326A a les propriétés physiques et chimiques suivantes :
   (1) forme et couleur :
      poudre incolore
   2) réaction aux couleurs :
      Positive :     réaction au sulfate de cérium, réaction à la vapeur d'iode réaction au chlorure ferrique-ferricyanure de potassium
      Négative :     réaction à la ninhydrine, réaction de Molish, réaction au chlorure ferrique, réaction d'Ehrlich, réaction de Pauli.
   (3) Solubilité :
      Soluble :          méthanol, éthanol
      Modérément soluble :    acétone, acétate d'éthyle
      Insoluble :         eau, chloroforme
   (4) Point de fusion : 187-190 °C
   (5) Rotation spécifique :
      $[\alpha]_D^{23}$ : -84° (C = 1,0; MeOH)
   (6) Spectre d'absorption de l'ultraviolet :

$$\lambda \, _{Max}^{MeOH} = 280 \text{ nm } (\varepsilon = 34.700)$$

   (7) Spectre d'absorption de l'infrarouge :

$$\upsilon \, _{Max}^{KBr} = 3300, 3050, 2950, 2920, 2860, 1730,$$
$$1650, 1610, 1560, 1540, 1530, 1510,$$
$$1440, 1380, 1340, 1280, 1240, 1170,$$
$$1110, 1080, 1060, 1040, 970, 920,$$
$$880, 860, 830 \text{ cm}^{-1}$$

   (8)

| Analyse élémentaire : | | | |
|---|---|---|---|
| Trouvé : | C 60,18, | H 6,61, | N 10,32 |
| Calc. pour $C_{54}H_{68}N_8O_{13}.2H_2O$ : | C 60,43, | H 6,76, | N 10,44 |

   (9) Chromatographie sur couche mince :

| Phase stationnaire | Solvant de développement | Valeur Rf |
|---|---|---|
| Plaque de gel de silice (Merck Art 5715 Plaque RP-18 | chloroforme-méthanol (5:1, V/V) méthanol-eau (8:2, V/V) | 0,38 0,46 |

   (10) Formule moléculaire : $C_{54}H_{68}N_8O_{13}$
   (11) Masse moléculaire :
      FAB-MS :    m/z 1037 (M + H)$^+$
   (12) Propriété de la substance :
      substance acide
   (13) Spectre de résonance magnétique nucléaire $^{13}C$ (100 MHz, CP$_3$OD) δ

| | |
|---|---|
| 175, 69 (s), | 174, 70 (s), |
| 173, 73 (s), | 173, 38 (s), |
| 172,89 (s), | 171,04 (s), |
| 170,45 (s), | 167,79 (s), |
| 167,15 (s), | 159,20 (s), |
| 140,05 (d), | 139,12 (s), |
| 138,71 (s), | 135,27 (d), |
| 134,85 (s), | 132,11 (d), |
| 132,03 (s), | 131,69 (d) x 2, |
| 130,70 (d), | 129,90 (d), |
| 129,61 (d) x 2, | 129,22 (d) x 2, |
| 128,55 (d), | 128,04 (d), |
| 127,99 (d), | 127,38 (d), |
| 126,09 (s), | 123,70 (d), |
| 115,63 (d) x 2, | 73,46 (d), |
| 71,34 (d), | 62,80 (t), |
| 59,53 (d), | 56,91 (d), |
| 56,76 (d), | 55,55 (d), |
| 53,64 (d), | 52,10 (d), |
| 39,85 (t), | 37,18 (t), |
| 37,09 (t), | 34,58 (q), |
| 31,37 (t), | 24,56 (d), |
| 23,63 (t), | 22,71 (q), |
| 22,52 (q), | 21,17 (q), |
| 17,19 (q), | 14,13 (q) |

(14) Spectre de résonance magnétique nucléaire $^1H$ (400 MHz, $CD_3OD$) $\delta$

| | |
|---|---|
| 7,80 | (1H, d, J = 8Hz), |
| 7,67 | (1H, d, J = 16Hz), |
| 7,45-7,14 | (9H, m), |
| 7,06 | (2H, d, J = 8Hz), |
| 6,83 | (1H, s), |
| 6,65 | (2H, d, J = 8Hz), |
| 6,59 | (1H, d, J = 12Hz), |
| 5,88 | (1H, dt, J = 12 et 7Hz), |
| 5,55 | (1H, m), |
| 5,35 | (1H, signal large), |
| 5,10 | (1H, dd, J = 3 et 9.5Hz), |
| 4,68 | (1H, d, J = 10Hz), |
| 4,55 | (1H, t, J = 6Hz), |
| 4,48 | (1H, dd, J = 3 et 12Hz), |
| 3,92 | (2H, d, J = 6Hz), |
| 3,70 | (1H, t, J = 7.5Hz), |
| 3,62 | (1H, m), |
| 3,46 | (1H, dd, J = 3 et 14Hz), |
| 2,94 | (1H, dd, J = 3 et 16Hz), |
| 2,89 | (3H, s), |
| ! 2,74 | (1H, dd, J = 9.5 et 16Hz), |
| 2,69 | (1H, dd, J = 12 et 14Hz), |
| 2,14 | (2H, m), |
| 1,5-1.4 | (2H, m), |
| 1,20 | (3H, d, J = 6Hz) |
| 1,08 | (3H, d, J = 6Hz), |
| 1,0-0.8 | (2H, m), |
| 0,91 | (3H, t, J = 7Hz), |
| 0,6 | (1H, m) |
| 0,53 | (3H, d, J = 6Hz), |
| 0,51 | (3H, d, J = 6Hz) |

(ii) WS-9326B a les propriétés physiques et chimiques suivantes :

(1) Forme et couleur : poudre amorphe incolore

(2) réaction aux couleurs :

Positive : réaction au sulfate de cérium, réaction à la vapeur diode

Négative : réaction à la ninhydrine

(3) Solubilité :

Soluble : méthanol

Modérément soluble : éthanol

Insoluble : eau, acétone, acétate d'éthyle, chloroforme

(4) Point de fusion : 165-170 °C (déc.)

(5) Rotation spécifique : $[\alpha]_D^{23}$ : -64° (C = 1,0; MeOH)

(6) Spectre de l'absorption de l'ultraviolet :

$$\lambda \, _{Max}^{MeOH} = 283nm \ (\varepsilon = 27.000)$$

(7) Formule moléculaire : $C_{54}H_{70}N_8O_{13}$

(8)

| Analyse élémentaire : | | | |
|---|---|---|---|
| Trouvé : | C 59,97, | H 6,87, | N 10,29 |
| Calc. pour : $C_{54}H_{70}N_8O_{13}.2H_2O$ : | C 60,32, | H 6,94, | N 10,42 |

(9) Masse moléculaire :

FAB-MS : m/z 1061,6 $(M + Na)^+$

(10) Chromatographie sur couche mince :

| Phase stationnaire | Solvant de développement | Valeur Rf |
|---|---|---|
| Plaque de gel de Silice (Merck Art 5715) | Chloroforme-méthanol (5:1, V/V) | 0,38 |
| Plaque RP-18 | méthanol-eau (8:2, V/V) | 0,25 |

(11) Spectre d'absorption de l'infrarouge :

$$\nu^{KBr}_{max} = 3300, 3050, 2950, 1735, 1660, 1530,$$
$$1510, 1450, 1400, 1380, 1340, 1260,$$
$$1220, 1080, 980, 920 \text{ cm}^{-1}$$

(12) Spectre de résonance magnétique nucléaire $13_C$ (100 MHz, $CD_3OD$) δ

| | |
|---|---|
| 174,99 (s), | 174,54 (s), |
| 173,60 (s), | 173,41 (s), |
| 173,30 (s), | 171,27 (s), |
| 170,74 (s), | 170,19 (s), |
| 168,69 (s), | 157,59 (s), |
| 140,53 (d), | 139,35 (s), |
| 139,18 (s), | 135,76 (d), |
| 134,17 (s), | 131,15 (d) x 2, |
| 130,93 (d), | 130,35 (d), |
| 129,88 (d) x 2, | 129,39 (d) x 2, |
| 128,70 (d), | 128,58 (s), |
| 128,13 (d), | 127,64 (d), |
| 127,53 (d), | 121,99 (d), |
| 116,45 (d) x 2, | 72,76 (d), |
| 70,82 (d), | 62,73 (t), |
| 62,67 (d), | 59,35 (d), |
| 56,33 (d) x 2, | 56,19 (d), |
| 53,36 (d), | 52,24 (d), |
| 40,24 (t), | 37,55 (t), |
| 37,08 (t), | 33,69 (t), |
| 31,57 (t), | 29,93 (q), |
| 24,61 (d), | 23,70 (q), |
| 23,59 (t), | 22,16 (q), |
| 21,36 (q), | 17,12 (q), |
| 14,23 (q) | |

(13) Spectre de résonance magnétique nucléaire $1_H$ : (400 MHz, $CD_3OP$) δ

91

| 7,86 | (1H, d, J = 16Hz), |
|---|---|
| 7,80 | (1H, br d, J = 8Hz), |
| 7,12-7,42 | (11H, m), |
| 6,77 | (2H, d, J = 8.5Hz), |
| 6,61 | (1H, d, J = 11.5Hz), |
| 5,88 | (1H, dt, J = 7.5 et 11.5Hz), |
| 5,08 | (1H, dd, J = 3.5 et 10Hz), |
| 5,04 | (1H, q, J = 6.5Hz), |
| 4,66 | (1H, dd, J = 3.5 et 13Hz), |
| 4,65 | (1H, d, J = 11.5Hz), |
| 4,56 | (1H, dd, J = 2.5 et 7Hz), |
| 4,48 | (1H, dd, J = 4.5 et 11Hz), |
| 4,46 | (1H; s), |
| 3,88 | (2H, m), |
| 3,64 | (2H, m), |
| 3,51 | (1H, dd, J = 3.5 et 14Hz), |
| 3,17 | (1H, dd, J = 4.5 et 14Hz), |
| 3,01 | (1H, dd, J = 11 et 14Hz), |
| 2,94 | (1H, dd, J = 3.5 et 16Hz), |
| 2,71 | (3H, s), |
| 2,71 | (1H, dd, J = 10 et 16Hz), |
| 2,64 | (1H, dd, J = 13 et 14Hz), |
| 2,04 | (2H, m), |
| 1,43 | (2H, m), |
| 1,28 | (2H, m), |
| 1,20 | (3H, d, J = 6Hz), |
| 0,95 | (3H, d, J = 6.5Hz), |
| 0,87 | (3H, t, J = 7.5Hz), |
| 0,53 | (1H, m), |
| 0,52 | (6H, d, J = 10.5Hz) |

qui comprend la mise en culture d'une souche produisant WS-9326A et/ou WS-9326B appartenant au genre Streptomyces dans un milieu nutritif et la récupération d'un composé choisi parmi WS-9326A et WS-9326B, ou un de leurs sels à partir du bouillon de culture obtenu.

2. Procédé selon la revendication 1, pour la production d'un composé choisi parmi :
parmi WS-9326A substitué avec 1 à 3 groupes acyle
et WS-9326B susbtitué avec 1 à 3 groupes acyle,
ou un de leurs sels,
qui comprend la mise en réaction d'un composé choisi parmi WS-9326A et WS-9326B, ou un de leurs sels avec un ou plusieurs agents d'acylation.

3. Procédé selon la revendication 1, pour la production de WS-9326A ou un de ses sels.

4. Procédé selon la revendication 1, pour la production de WS-9326B ou un de ses sels.

5. Procédé selon la revendication 1, pour la production de WS-9326A susbtitué par 1 à 3 groupes acyle ou ses sels.

6. Procédé selon la revendication 5, dans lequel le groupe acyle est un groupe alkanoyl inférieur.

7. Procédé selon la revendication 6, pour la production de triacétyl-WS-9326A ayant les prorpiétés physiques et chimiques suivantes :
(1) Forme et couleur : poudre incolore
(2) réaction aux couleurs :
Positive : réaction au sulfate de cérium, réaction à l'acide sulfurique réaction à la vapeur d'iode

Négative : réaction à la ninhydrine

(3) Solubilité :

Soluble : méthanol, sulfoxyde de diméthyle

Modérément soluble : chloroforme, éther diéthylique

Insoluble : n-héxane

(4) Point de fusion : 141-143°C

(5) Rotation spécifique :$[\alpha]_D^{23}$ : -122° (C = 1,0; MeOH)

(6) Spectre d'absorption de l'ultraviolet :

$$\lambda_{Max}^{MeOH} = 283 \text{ nm } (\varepsilon = 32.000)$$

(7) Formule moléculaire : $C_{60}H_{74}N_8O_{16}$

(8)

| Analyse élémentaire : | | | |
|---|---|---|---|
| Trouvé : | C 60,19, | H 6,42, | N 9,27 |
| Calc. pour : $C_{60}H_{74}N_8O_{16} \cdot 2H_2O$ : | C 60,09, | H 6,56, | N 9,34 |

(9) Masse moléculaire :

FAB-MS : m/z 1163,6 $(M+H)^+$

(10) Chromatographie sur couche mince :

| Phase stationnaire | Solvant de développement | Valeur Rf |
|---|---|---|
| Plaque de gel de Silice (Merck Art 5715) | Chloroformeméthanol (10:1, V/V) acétate d'éthyle | 0,50 0,12 |

(11) Spectre d'absorption de l'infrarouge :

$$\nu_{max}^{KBr} = 3350, 3020, 2950, 2920, 2850, 1730,$$
$$1650, 1520, 1440, 1360, 1230, 1200,$$
$$1160, 1100, 1060, 1040, 910 \text{ cm}^{-1}$$

(12) Propriété de la substance :

substance neutre

(13) Spectre de résonance magnétique nucléaire $13_C$ (100 MHz, $CDC\ell_3$-$CD_3OH$ (10:1)) $\delta$

| | |
|---|---|
| 174,20 (s), | 173,23 (s), |
| 173,06 (s), | 171,32 (s), |
| 171,02 (s), | 170,84 (s), |
| 169,79 (s), | 169,59 (s), |
| 169,55 (s), | 168,52 (s), |
| 167,03 (s), | 166,36 (s), |
| 151,02 (s), | 140,74 (d), |
| 138,82 (s), | 138,74 (s), |
| 137,12 (s), | 135,23 (d), |
| 133,75 (s), | 131,31 (s), |
| 130,20 (d), | 129,96 (d) x 2, |
| 129,34 (d), | 129,21 (d) x 2, |
| 128,56 (d) x 2, | 127,24 (d), |
| 126,95 (d), | 126,74 (d), |
| 126,63 (d), | 126,50 (d), |
| 122,10 (d) x 2, | 121,29 (d), |
| 70,99 (d), | 69,22 (d), |
| 63,73 (t), | 58,13 (d), |
| 56,10 (d), | 53,22 (d), |
| 52,66 (d), | 52,18 (d), |
| 49,93 (d), | 39,75 (t), |
| 39,39 (q), | 39,06 (t), |
| 35,57 (t), | 30,65 (t), |
| 24,26 (d), | 23,15 (q), |
| 22,79 (t), | 21,42 (q), |
| 21,21 (q), | 20,99 (q), |
| 20,83 (q), | 17,05 (q), |
| 16,18 (q), | 13,82 (q) |

(14) Spectre de résonance magnétique nucléaire $^1$H (400 MHz, CDC$\ell_3$-CD$_3$OH (10:1)) $\delta$

```
8,25        (1H, d, J=8Hz),
8,02        (1H, d, J=8Hz),
7,88        (1H, d, J=16Hz),
7,86        (1H, d, J=8Hz),
7,70        (1H, d, J=6Hz),
7,61        (1H, d, J=8Hz),
7,45        (1H, d, J=7Hz),
7,32-7,15 (6H, m),
7,03        (2H, d, J=8Hz),
7,00-6,94 (3H, m),
6,88-6,79 (4H, m),
6,70        (1H, s),
6,49        (1H, d, J=12Hz),
5,76        (1H, dt, J=12 et 7.5Hz),
5,54        (1H, broad s),
5,50-5,45 (2H, m),
4,93        (1H, m),
4,75        (1H, m)
4,65-4,56 (2H, m),
4,46        (1H, dd, J=6 et 11Hz),
4,31        (1H, t, J=6Hz),
4,22        (1H, m),
4,18        (1H, dd, J=8 et 11Hz),
3,56        (3H, s),
2,90        (1H, dd, J=6 et 16Hz),
2,85-2,80 (2H, m),
2,56        (1H, dd, J=4 et 16Hz),
2,26        (3H, s),
2,00        (3H, s),
1,96-1,89 (2H, m),
1,85        (3H, s),
1,58        (1H, m),
1,35        (3H, d, J=6Hz),
1,32-1,20 (3H, m),
1,07        (3H, d, J=6Hz),
0,84        (1H, m),
0,72        (3H, d, J=6Hz),
0,71        (3H, t, J=7.5Hz),
0,65        (3H, d, J=6Hz)
```

8. Modification du procédé de l'une quelconques des revendicationS 1 à 7, qui est caractérisé en ce qu'on met un composé préparé par le procédé de l'une quelconque des revendications 1 à 7 ou un de ses sels pharmaceutiquement acceptables sous forme acceptable par mélange ou présentation dudit composé avec un diluant ou un support pharmaceutiquement acceptable.

9. Culture biologiquement pure du microorganisme Streptomyces violaceoniger No. 9326 (FERM BP-1667)

Figure 1

Spectrum of Infrared Absorption of WS-9326A in KBr

97

Figure 2

Spectrum of $^{13}$C Nuclear Magnetic Resonance
of WS-9326A in $CD_3OD$

190  180  170  160  150  140  130  120  110  100  90  80  70  60  50  40  30  20  10  0
PPM

Figure 3

Spectrum of $^1$H Nuclear Magnetic
Resonance of WS-9326A in $CD_3OD$

Figure 4

Spectrum of $^{13}$C Nuclear Magnetic Resonance
of triacetyl-WS-9326A in CDCl$_3$-CD$_3$OH (10:1)

EP 0 327 009 B1

Figure 5

Spectrum of $^{1}$H Nuclear Magnetic Resonance
of triacetyl-WS-9326A in $CDCl_3$-$CD_3OH$ (10:1)

9.5  9.0  8.5  8.0  7.5  7.0  6.5  6.0  5.5  5.0  4.5  4.0  3.5  3.0  2.5  2.0  1.5  1.0  .5  0.0
PPM

EP 0 327 009 B1

Figure 6

Spectrum of $^{13}$C Nuclear Magnetic Resonance

of WS-9326B in $CD_3OD$

Figure 7

Spectrum of ${}^{1}$H Nuclear Magnetic Resonance of WS-9326B in CD$_3$OD

Figure 8

Spectrum of $^1$H Nuclear Magnetic Resonance

of monoacetyl-WS-9326A in $CDCl_3$-$CD_3OD$ (5:1)

Figure 9

Spectrum of $^1$H Nuclear Magnetic Resonance
of diacetyl-WS-9326A in $CDCl_3$-$CD_3OD$ (5:1)